# EUROPEAN PATENT APPLICATION

(11) **EP 4 063 387 A1**
(43) Date of publication of application: **28.09.2022**
(21) Application number: 20889117.6
(22) Date of filing: 20.11.2020
(51) Int. Cl.: C07K 16/28, C07K 16/30, A61K 39/395, C12P 21/04

(54) **PSMA ANTIBODY AND USE THEREOF**

(30) Priority: 22.11.2019 CN 201911155025
(71) Applicant: Nantong Yichen Biopharma. Co. Ltd., Nantong, Jiangsu 226001 (CN)
(72) Inventor: WANG, Feng, Shanghai 200233 (CN); ZHENG, Huayang, Shanghai 200233 (CN); ZHANG, Yuhan, Shanghai 200233 (CN)
(74) Representative: Biggi, Cristina
(86) International application number: PCT/CN2020/130511
(87) International publication number: WO 2021/098834

(57) **Abstract**

The present disclosure relates to an antibody fragment specifically binding to PSMA, particularly to a single-chain Fv (scFv), a bispecific antibody containing the scFv, and a chimeric antigen receptor (CAR) and the preparation and the use thereof.

SEQUENCE LISTING

<110>  SHANGHAI YICHEN BIOMED CO.LTD
       WANG, Feng
       ZHENG, Huayang
       ZHANG, Yuhan

<120>  PSMA ANTIBODY AND USE THEREOF

<130>  PN185904YCYY

<150>  201911155025.8
<151>  2019-11-22

<160>  68

<170>  PatentIn version 3.5

<210>  1
<211>  345
<212>  DNA
<213>  Artificial Sequence

<220>
<223>  Synthesized

<400>  1
gaggtgcagc tggtgcagag cggccccgag gtgaagaagc ccggcgccac cgtgaagatc      60

agctgcaaga ccagcggcta caccttcacc gagtacacca tccactgggt gaagcaggcc     120

cccggcaagg gcctggagtg gatcggcaac atcaacccca acaacggcgg caccacctac     180

aaccagaagt tcgaggacaa ggccaccctg accgtggaca agagcaccga caccgcctac     240

atggagctga gcagcctgcg cagcgaggac accgccgtgt actactgcgc cgccggctgg     300

aacttcgact actggggcca gggcacccta cttacagtaa gtagc                     345


<210>  2
<211>  115
<212>  PRT
<213>  Artificial Sequence

<220>
<223>  Synthesized

<400>  2

Glu Val Gln Leu Val Gln Ser Gly Pro Glu Val Lys Lys Pro Gly Ala
1               5                   10                  15


Thr Val Lys Ile Ser Cys Lys Thr Ser Gly Tyr Thr Phe Thr Glu Tyr
            20                  25                  30


Thr Ile His Trp Val Lys Gln Ala Pro Gly Lys Gly Leu Glu Trp Ile
        35                  40                  45


Gly Asn Ile Asn Pro Asn Asn Gly Gly Thr Thr Tyr Asn Gln Lys Phe

50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser
        115

<210>  3
<211>  321
<212>  DNA
<213>  Artificial Sequence

<220>
<223>  Synthesized

<400>  3
   gacatccaga tgacccagag ccccagcagc ctgagcacca gcgtgggcga ccgcgtgacc      60
   ctgacctgca aggccagcca ggacgtgggc accgccgtgg actggtacca gcagaagccc     120
   ggccccagcc ccaagctgct gatctactgg gccagcaccc gccacaccgg catccccagc     180
   cgcttcagcg gcagcggcag cggcaccgac ttcaccctga ccatcagcag cctgcagccc     240
   gaggacttcg ccgactacta ctgccagcag tacaacagct accccctgac cttcggcccc     300
   ggcaccaagg tggacatcaa g                                               321

<210>  4
<211>  107
<212>  PRT
<213>  Artificial Sequence

<220>
<223>  Synthesized

<400>  4

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Pro Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys
            100                 105

<210>  5
<211>  321
<212>  DNA
<213>  Artificial Sequence

<220>
<223>  Synthesized

<400>  5
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggcctcagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa g                                               321

<210>  6
<211>  107
<212>  PRT
<213>  Artificial Sequence

<220>
<223>  Synthesized

<400>  6

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Leu Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys
            100                 105

<210>  7
<211>  321
<212>  DNA
<213>  Artificial Sequence

<220>
<223>  Synthesized

<400>  7
   gacatccaga tgacccagag ccccagcagc ctgagcacca gcgtgggcga ccgcgtgacc      60
   ctgacctgca aggccagcca ggacgtgggc accgccgtgg actggtacca gcagaagccc     120
   ggccacagcc ccaagctgct gatctactgg gccagcaccc gccacaccgg catccccagc     180
   cgcttcagcg gcagcggcag cggcaccgac ttcaccctga ccatcagcag cctgcagccc     240
   gaggacttcg ccgactacta ctgccagcag tacaacagct accccctgac cttcggcccc     300
   ggcaccaagg tggacatcaa g                                               321

<210>  8
<211>  107
<212>  PRT
<213>  Artificial Sequence

<220>
<223>  Synthesized

<400>  8

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly His Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys
            100                 105

<210>  9
<211>  321
<212>  DNA
<213>  Artificial Sequence

<220>
<223>  Synthesized

<400>  9
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggcaccagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa g                                               321

<210>  10
<211>  107
<212>  PRT
<213>  Artificial Sequence

<220>
<223>  Synthesized

<400>  10

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Thr Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu

85                  90                  95

Thr Phe Gly Pro Gly Thr Lys Val Asp Ile Lys
            100                 105

<210>  11
<211>  321
<212>  DNA
<213>  Artificial Sequence

<220>
<223>  Synthesized

<400>  11
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggctccagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa g                                               321

<210>  12
<211>  107
<212>  PRT
<213>  Artificial Sequence

<220>
<223>  Synthesized

<400>  12

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Ser Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys
            100                 105

<210>  13
<211>  321
<212>  DNA
<213>  Artificial Sequence

<220>
<223>  Synthesized

<400>  13
gacatccaga tgacccagag ccccagcagc ctgagcacca gcgtgggcga ccgcgtgacc        60

ctgacctgca aggccagcca ggacgtgggc accgccgtgg actggtacca gcagaagccc       120

ggccagagcc ccaagctgct gatctactgg gccagcaccc gccacaccgg catccccagc       180

cgcttcagcg gcagcggcag cggcaccgac ttcaccctga ccatcagcag cctgcagccc       240

gaggacttcg ccgactacta ctgccagcag tacaacagct accccctgac cttcggcccc       300

ggcaccaagg tggacatcaa g                                                 321

<210>  14
<211>  107
<212>  PRT
<213>  Artificial Sequence

<220>
<223>  Synthesized

<400>  14

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Gln Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys
            100                 105

<210>  15
<211>  711
<212>  DNA
<213>  Artificial Sequence

<220>
<223>  Synthesized

<400>  15
gaggtgcagc tggtgcagag cggccccgag gtgaagaagc ccggcgccac cgtgaagatc      60

agctgcaaga ccagcggcta caccttcacc gagtacacca tccactgggt gaagcaggcc     120

cccggcaagg gcctggagtg gatcggcaac atcaacccca acaacggcgg caccacctac     180

aaccagaagt tcgaggacaa ggccaccctg accgtggaca agagcaccga caccgcctac     240

atggagctga gcagcctgcg cagcgaggac accgccgtgt actactgcgc cgccggctgg     300

aacttcgact actggggcca gggcacccta cttacagtaa gtagcggtgg cggtggctcg     360

ggcggtggtg ggtcgggtgg cggcggatct gacatccaga tgacccagag ccccagcagc     420

ctgagcacca gcgtgggcga ccgcgtgacc ctgacctgca aggccagcca ggacgtgggc     480

accgccgtgg actggtacca gcagaagccc ggccccagcc ccaagctgct gatctactgg     540

gccagcaccc gccacaccgg catccccagc cgcttcagcg gcagcggcag cggcaccgac     600

ttcaccctga ccatcagcag cctgcagccc gaggacttcg ccgactacta ctgccagcag     660

tacaacagct accccctgac cttcggcccc ggcaccaagg tggacatcaa g               711


<210>  16
<211>  237
<212>  PRT
<213>  Artificial Sequence

<220>
<223>  Synthesized

<400>  16

Glu Val Gln Leu Val Gln Ser Gly Pro Glu Val Lys Lys Pro Gly Ala
1               5                   10                  15


Thr Val Lys Ile Ser Cys Lys Thr Ser Gly Tyr Thr Phe Thr Glu Tyr
            20                  25                  30


Thr Ile His Trp Val Lys Gln Ala Pro Gly Lys Gly Leu Glu Trp Ile
        35                  40                  45


Gly Asn Ile Asn Pro Asn Asn Gly Gly Thr Thr Tyr Asn Gln Lys Phe
    50                  55                  60


Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Thr Ser
    130                 135                 140

Val Gly Asp Arg Val Thr Leu Thr Cys Lys Ala Ser Gln Asp Val Gly
145                 150                 155                 160

Thr Ala Val Asp Trp Tyr Gln Gln Lys Pro Gly Pro Ser Pro Lys Leu
                165                 170                 175

Leu Ile Tyr Trp Ala Ser Thr Arg His Thr Gly Ile Pro Ser Arg Phe
            180                 185                 190

Ser Gly Ser Gly Ser Gly Thr Asp Phe Thr Leu Thr Ile Ser Ser Leu
        195                 200                 205

Gln Pro Glu Asp Phe Ala Asp Tyr Tyr Cys Gln Gln Tyr Asn Ser Tyr
    210                 215                 220

Pro Leu Thr Phe Gly Pro Gly Thr Lys Val Asp Ile Lys
225                 230                 235

<210>  17
<211>  711
<212>  DNA
<213>  Artificial Sequence

<220>
<223>  Synthesized

<400>  17
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccccagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag c 711

<210> 18
<211> 237
<212> PRT
<213> Artificial Sequence

<220>
<223> Synthesized

<400> 18

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Pro Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu
        195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210>  19
<211>  711
<212>  DNA
<213>  Artificial Sequence

<220>
<223>  Synthesized

<400>  19
gaggtgcagc tggtgcagag cggccccgag gtgaagaagc ccggcgccac cgtgaagatc      60

agctgcaaga ccagcggcta caccttcacc gagtacacca tccactgggt gaagcaggcc     120

cccggcaagg gcctggagtg gatcggcaac atcaacccca acaacggcgg caccacctac     180

aaccagaagt tcgaggacaa ggccaccctg accgtggaca agagcaccga caccgcctac     240

atggagctga gcagcctgcg cagcgaggac accgccgtgt actactgcgc cgccggctgg     300

aacttcgact actggggcca gggcacccta cttacagtaa gtagcggtgg cggtggctcg     360

ggcggtggtg ggtcgggtgg cggcggatct gacatccaga tgacccagag ccccagcagc     420

ctgagcacca gcgtgggcga ccgcgtgacc ctgacctgca aggccagcca ggacgtgggc     480

accgccgtgg actggtacca gcagaagccc ggcctcagcc ccaagctgct gatctactgg     540

gccagcaccc gccacaccgg catccccagc cgcttcagcg gcagcggcag cggcaccgac     600

ttcaccctga ccatcagcag cctgcagccc gaggacttcg ccgactacta ctgccagcag     660

tacaacagct accccctgac cttcggcccc ggcaccaagg tggacatcaa g             711

<210>  20
<211>  237
<212>  PRT
<213>  Artificial Sequence

<220>
<223>  Synthesized

<400>  20

Glu Val Gln Leu Val Gln Ser Gly Pro Glu Val Lys Lys Pro Gly Ala
1               5                   10                  15

Thr Val Lys Ile Ser Cys Lys Thr Ser Gly Tyr Thr Phe Thr Glu Tyr
            20                  25                  30

Thr Ile His Trp Val Lys Gln Ala Pro Gly Lys Gly Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Thr Ser
    130                 135                 140

Val Gly Asp Arg Val Thr Leu Thr Cys Lys Ala Ser Gln Asp Val Gly
145                 150                 155                 160

Thr Ala Val Asp Trp Tyr Gln Gln Lys Pro Gly Leu Ser Pro Lys Leu
                165                 170                 175

Leu Ile Tyr Trp Ala Ser Thr Arg His Thr Gly Ile Pro Ser Arg Phe
            180                 185                 190

Ser Gly Ser Gly Ser Gly Thr Asp Phe Thr Leu Thr Ile Ser Ser Leu
        195                 200                 205

Gln Pro Glu Asp Phe Ala Asp Tyr Tyr Cys Gln Gln Tyr Asn Ser Tyr
    210                 215                 220

Pro Leu Thr Phe Gly Pro Gly Thr Lys Val Asp Ile Lys
225                 230                 235

<210>  21
<211>  711

<212>  DNA
<213>  Artificial Sequence

<220>
<223>  Synthesized

<400>  21
   gaggtgcagc tggtgcagag cggccccgag gtgaagaagc ccggcgccac cgtgaagatc      60
   agctgcaaga ccagcggcta caccttcacc gagtacacca tccactgggt gaagcaggcc     120
   cccggcaagg gcctggagtg gatcggcaac atcaacccca acaacggcgg caccacctac     180
   aaccagaagt tcgaggacaa ggccaccctg accgtggaca agagcaccga caccgcctac     240
   atggagctga gcagcctgcg cagcgaggac accgccgtgt actactgcgc cgccggctgg     300
   aacttcgact actggggcca gggcacccta cttacagtaa gtagcggtgg cggtggctcg     360
   ggcggtggtg ggtcgggtgg cggcggatct gacatccaga tgacccagag ccccagcagc     420
   ctgagcacca gcgtgggcga ccgcgtgacc ctgacctgca aggccagcca ggacgtgggc     480
   accgccgtgg actggtacca gcagaagccc ggcctcagcc ccaagctgct gatctactgg     540
   gccagcaccc gccacaccgg catccccagc cgcttcagcg gcagcggcag cggcaccgac     600
   ttcaccctga ccatcagcag cctgcagccc gaggacttcg ccgactacta ctgccagcag     660
   tacaacagct accccctgac cttcggcccc ggcaccaagg tggacatcaa g             711


<210>  22
<211>  237
<212>  PRT
<213>  Artificial Sequence

<220>
<223>  Synthesized

<400>  22

Glu Val Gln Leu Val Gln Ser Gly Pro Glu Val Lys Lys Pro Gly Ala
1               5                   10                  15


Thr Val Lys Ile Ser Cys Lys Thr Ser Gly Tyr Thr Phe Thr Glu Tyr
            20                  25                  30


Thr Ile His Trp Val Lys Gln Ala Pro Gly Lys Gly Leu Glu Trp Ile
        35                  40                  45


Gly Asn Ile Asn Pro Asn Asn Gly Gly Thr Thr Tyr Asn Gln Lys Phe
    50                  55                  60


Glu Asp Lys Ala Thr Leu Thr Val Asp Lys Ser Thr Asp Thr Ala Tyr
65                  70                  75                  80


Met Glu Leu Ser Ser Leu Arg Ser Glu Asp Thr Ala Val Tyr Tyr Cys

85                  90                  95

Ala Ala Gly Trp Asn Phe Asp Tyr Trp Gly Gln Gly Thr Leu Leu Thr
            100                 105                 110

Val Ser Ser Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Thr Ser
    130                 135                 140

Val Gly Asp Arg Val Thr Leu Thr Cys Lys Ala Ser Gln Asp Val Gly
145                 150                 155                 160

Thr Ala Val Asp Trp Tyr Gln Gln Lys Pro Gly Leu Ser Pro Lys Leu
                165                 170                 175

Leu Ile Tyr Trp Ala Ser Thr Arg His Thr Gly Ile Pro Ser Arg Phe
            180                 185                 190

Ser Gly Ser Gly Ser Gly Thr Asp Phe Thr Leu Thr Ile Ser Ser Leu
        195                 200                 205

Gln Pro Glu Asp Phe Ala Asp Tyr Tyr Cys Gln Gln Tyr Asn Ser Tyr
    210                 215                 220

Pro Leu Thr Phe Gly Pro Gly Thr Lys Val Asp Ile Lys
225                 230                 235

<210>  23
<211>  711
<212>  DNA
<213>  Artificial Sequence

<220>
<223>  Synthesized

<400>  23
   gaggtgcagc tggtgcagag cggccccgag gtgaagaagc ccggcgccac cgtgaagatc      60
   agctgcaaga ccagcggcta caccttcacc gagtacacca tccactgggt gaagcaggcc     120
   cccggcaagg gcctggagtg gatcggcaac atcaacccca acaacggcgg caccacctac     180
   aaccagaagt tcgaggacaa ggccaccctg accgtggaca agagcaccga caccgcctac     240
   atggagctga gcagcctgcg cagcgaggac accgccgtgt actactgcgc cgccggctgg     300
   aacttcgact actggggcca gggcacccta cttacagtaa gtagcggtgg cggtggctcg     360
   ggcggtggtg ggtcgggtgg cggcggatct gacatccaga tgacccagag ccccagcagc     420
   ctgagcacca gcgtgggcga ccgcgtgacc ctgacctgca aggccagcca ggacgtgggc     480
   accgccgtgg actggtacca gcagaagccc ggcaccagcc ccaagctgct gatctactgg 540
   gccagcaccc gccacaccgg catccccagc cgcttcagcg gcagcggcag cggcaccgac 600
   ttcaccctga ccatcagcag cctgcagccc gaggacttcg ccgactacta ctgccagcag 660
   tacaacagct accccctgac cttcggcccc ggcaccaagg tggacatcaa g 711

<210> 24
<211> 237
<212> PRT
<213> Artificial Sequence

<220>
<223> Synthesized

<400> 24

Glu Val Gln Leu Val Gln Ser Gly Pro Glu Val Lys Lys Pro Gly Ala
1               5                   10                  15

Thr Val Lys Ile Ser Cys Lys Thr Ser Gly Tyr Thr Phe Thr Glu Tyr
            20                  25                  30

Thr Ile His Trp Val Lys Gln Ala Pro Gly Lys Gly Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Thr Ser
    130                 135                 140

Val Gly Asp Arg Val Thr Leu Thr Cys Lys Ala Ser Gln Asp Val Gly
145                 150                 155                 160

Thr Ala Val Asp Trp Tyr Gln Gln Lys Pro Gly Thr Ser Pro Lys Leu
                165                 170                 175

Leu Ile Tyr Trp Ala Ser Thr Arg His Thr Gly Ile Pro Ser Arg Phe
            180                 185                 190

Ser Gly Ser Gly Ser Gly Thr Asp Phe Thr Leu Thr Ile Ser Ser Leu
        195                 200                 205

Gln Pro Glu Asp Phe Ala Asp Tyr Tyr Cys Gln Gln Tyr Asn Ser Tyr
    210                 215                 220

Pro Leu Thr Phe Gly Pro Gly Thr Lys Val Asp Ile Lys
225                 230                 235

<210>  25
<211>  711
<212>  DNA
<213>  Artificial Sequence

<220>
<223>  Synthesized

<400>  25
gaggtgcagc tggtgcagag cggccccgag gtgaagaagc ccggcgccac cgtgaagatc      60

agctgcaaga ccagcggcta caccttcacc gagtacacca tccactgggt gaagcaggcc     120

cccggcaagg gcctggagtg gatcggcaac atcaacccca acaacggcgg caccacctac     180

aaccagaagt tcgaggacaa ggccaccctg accgtggaca agagcaccga caccgcctac     240

atggagctga gcagcctgcg cagcgaggac accgccgtgt actactgcgc cgccggctgg     300

aacttcgact actggggcca gggcacccta cttacagtaa gtagcggtgg cggtggctcg     360

ggcggtggtg ggtcgggtgg cggcggatct gacatccaga tgacccagag ccccagcagc     420

ctgagcacca gcgtgggcga ccgcgtgacc ctgacctgca aggccagcca ggacgtgggc     480

accgccgtgg actggtacca gcagaagccc ggctccagcc ccaagctgct gatctactgg     540

gccagcaccc gccacaccgg catccccagc cgcttcagcg gcagcggcag cggcaccgac     600

ttcaccctga ccatcagcag cctgcagccc gaggacttcg ccgactacta ctgccagcag     660

tacaacagct accccctgac cttcggcccc ggcaccaagg tggacatcaa g             711

<210>  26
<211>  237
<212>  PRT
<213>  Artificial Sequence

<220>
<223>  Synthesized

<400>  26

Glu Val Gln Leu Val Gln Ser Gly Pro Glu Val Lys Lys Pro Gly Ala
1               5                   10                  15

Thr Val Lys Ile Ser Cys Lys Thr Ser Gly Tyr Thr Phe Thr Glu Tyr
            20                  25                  30

Thr Ile His Trp Val Lys Gln Ala Pro Gly Lys Gly Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Thr Ser
    130                 135                 140

Val Gly Asp Arg Val Thr Leu Thr Cys Lys Ala Ser Gln Asp Val Gly
145                 150                 155                 160

Thr Ala Val Asp Trp Tyr Gln Gln Lys Pro Gly Ser Ser Pro Lys Leu
                165                 170                 175

Leu Ile Tyr Trp Ala Ser Thr Arg His Thr Gly Ile Pro Ser Arg Phe
            180                 185                 190

Ser Gly Ser Gly Ser Gly Thr Asp Phe Thr Leu Thr Ile Ser Ser Leu
        195                 200                 205

Gln Pro Glu Asp Phe Ala Asp Tyr Tyr Cys Gln Gln Tyr Asn Ser Tyr
    210                 215                 220

Pro Leu Thr Phe Gly Pro Gly Thr Lys Val Asp Ile Lys
225                 230                 235

<210>  27
<211>  711
<212>  DNA
<213>  Artificial Sequence

<220>
<223>  Synthesized

<400>  27
gaggtgcagc tggtgcagag cggccccgag gtgaagaagc ccggcgccac cgtgaagatc      60

agctgcaaga ccagcggcta caccttcacc gagtacacca tccactgggt gaagcaggcc     120

cccggcaagg gcctggagtg gatcggcaac atcaacccca acaacggcgg caccacctac     180

aaccagaagt tcgaggacaa ggccaccctg accgtggaca agagcaccga caccgcctac     240

atggagctga gcagcctgcg cagcgaggac accgccgtgt actactgcgc cgccggctgg     300

aacttcgact actggggcca gggcacccta cttacagtaa gtagcggtgg cggtggctcg     360

ggcggtggtg ggtcgggtgg cggcggatct gacatccaga tgacccagag ccccagcagc     420

ctgagcacca gcgtgggcga ccgcgtgacc ctgacctgca aggccagcca ggacgtgggc     480

accgccgtgg actggtacca gcagaagccc ggccagagcc ccaagctgct gatctactgg     540

gccagcaccc gccacaccgg catccccagc cgcttcagcg gcagcggcag cggcaccgac     600

ttcaccctga ccatcagcag cctgcagccc gaggacttcg ccgactacta ctgccagcag     660

tacaacagct accccctgac cttcggcccc ggcaccaagg tggacatcaa g               711

<210>  28
<211>  237
<212>  PRT
<213>  Artificial Sequence

<220>
<223>  Synthesized

<400>  28

Glu Val Gln Leu Val Gln Ser Gly Pro Glu Val Lys Lys Pro Gly Ala
1               5                   10                  15

Thr Val Lys Ile Ser Cys Lys Thr Ser Gly Tyr Thr Phe Thr Glu Tyr
            20                  25                  30

Thr Ile His Trp Val Lys Gln Ala Pro Gly Lys Gly Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Thr Ser
    130                 135                 140

Val Gly Asp Arg Val Thr Leu Thr Cys Lys Ala Ser Gln Asp Val Gly
145                 150                 155                 160

Thr Ala Val Asp Trp Tyr Gln Gln Lys Pro Gly Gln Ser Pro Lys Leu
                165                 170                 175

Leu Ile Tyr Trp Ala Ser Thr Arg His Thr Gly Ile Pro Ser Arg Phe
            180                 185                 190

Ser Gly Ser Gly Ser Gly Thr Asp Phe Thr Leu Thr Ile Ser Ser Leu
        195                 200                 205

Gln Pro Glu Asp Phe Ala Asp Tyr Tyr Cys Gln Gln Tyr Asn Ser Tyr
    210                 215                 220

Pro Leu Thr Phe Gly Pro Gly Thr Lys Val Asp Ile Lys
225                 230                 235

<210>  29
<211>  711
<212>  DNA
<213>  Artificial Sequence

<220>
<223>  Synthesized

<400>  29
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccagagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag c 711

<210> 30
<211> 237
<212> PRT
<213> Artificial Sequence

<220>
<223> Synthesized

<400> 30

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Gln Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu
        195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210>  31
<211>  711
<212>  DNA
<213>  Artificial Sequence

<220>
<223>  Synthesized

<400>  31
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggctccagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc     600

gcctacatgg agctgagcag cctgcgcagc gaggacaccg ccgtgtacta ctgcgccgcc     660

ggctggaact tcgactactg gggccagggc accctactta cagtaagtag c              711

<210>  32
<211>  237
<212>  PRT
<213>  Artificial Sequence

<220>
<223>  Synthesized

<400>  32

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala

20                  25                  30

Val Asp Trp Tyr Gln Gln Lys Pro Gly Ser Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu
        195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210>  33
<211>  711
<212>  DNA
<213>  Artificial Sequence

<220>
<223>  Synthesized

<400>  33
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggcaccagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc     600

gcctacatgg agctgagcag cctgcgcagc gaggacaccg ccgtgtacta ctgcgccgcc     660

ggctggaact tcgactactg gggccagggc accctactta cagtaagtag c              711

<210>  34
<211>  237
<212>  PRT
<213>  Artificial Sequence

<220>
<223>  Synthesized

<400>  34

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Thr Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu
        195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210>  35
<211>  711
<212>  DNA
<213>  Artificial Sequence

<220>
<223>  Synthesized

<400>  35
   gacatccaga tgacccagag ccccagcagc ctgagcacca gcgtgggcga ccgcgtgacc      60
   ctgacctgca aggccagcca ggacgtgggc accgccgtgg actggtacca gcagaagccc     120
   ggccacagcc ccaagctgct gatctactgg gccagcaccc gccacaccgg catccccagc     180
   cgcttcagcg gcagcggcag cggcaccgac ttcaccctga ccatcagcag cctgcagccc     240
   gaggacttcg ccgactacta ctgccagcag tacaacagct accccctgac cttcggcccc      300
   ggcaccaagg tggacatcaa gggtggcggt ggctcgggcg gtggtgggtc gggtggcggc     360
   ggatctgagg tgcagctggt gcagagcggc cccgaggtga agaagcccgg cgccaccgtg     420
   aagatcagct gcaagaccag cggctacacc ttcaccgagt acaccatcca ctgggtgaag     480
   caggcccccg gcaagggcct ggagtggatc ggcaacatca accccaacaa cggcggcacc     540
   acctacaacc agaagttcga ggacaaggcc accctgaccg tggacaagag caccgacacc     600
   gcctacatgg agctgagcag cctgcgcagc gaggacaccg ccgtgtacta ctgcgccgcc     660
   ggctggaact tcgactactg gggccagggc accctactta cagtaagtag c                711

<210>  36
<211>  237
<212>  PRT
<213>  Artificial Sequence

<220>
<223>  Synthesized

<400>  36

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly His Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu

195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210>  37
<211>  711
<212>  DNA
<213>  Artificial Sequence

<220>
<223>  Synthesized

<400>  37
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggcctcagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc     600

gcctacatgg agctgagcag cctgcgcagc gaggacaccg ccgtgtacta ctgcgccgcc     660

ggctggaact tcgactactg gggccagggc accctactta cagtaagtag c              711

<210>  38
<211>  237
<212>  PRT
<213>  Artificial Sequence

<220>
<223>  Synthesized

<400>  38

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Leu Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu
        195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210> 39
<211> 375
<212> DNA
<213> Artificial Sequence

<220>
<223> Synthesized

<400> 39
gaggtgcagc tggtggagtc tgggggaggc ttggtccagc ctggggggtc cctgcgtctc 60

tcctgtgcag cctctggatt caccttcaat acctacgcca tgaactgggt ccgccaggct     120

ccgggaaagg gtttggaatg ggttgctcgc attcgcagta aatataataa ttatgcaaca     180

tattatgccg attcagtgaa agaccgtttc accatctccc gcgatgattc aaagaacacg     240

ctctatctgc aaatgaacag cctgcgcgct gaggacacgg ccgtgtatta ctgcgtgcgt     300

catggcaact ttggtaattc ttatgtgagc tggttcgcct actggggcca gggaaccctg     360

gtcaccgtca gctct                                                      375

<210>  40
<211>  125
<212>  PRT
<213>  Artificial Sequence

<220>
<223>  Synthesized

<400>  40

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210>  41
<211>  330
<212>  DNA
<213>  Artificial Sequence

<220>
<223>  Synthesized

<400>  41
caagctgttg tgacccaaga accgagtctg accgtgtctc cgggcggcac cgttacgctg      60

acctgtcgct cctctaccgg cgcagtcacc acgagcaact atgcaaattg ggtgcagcaa     120

aaaccggatc atctgtttcg tggcctgatt ggcggtacga acaaacgtgc gccggggacc     180

ccggcacgtt tttcgggcag cctgctgggc gataaagcag cactgaccat cagtggtgcg     240

cagccggaag atgaagcaga atattactgc gctctgtggt atagcaacct gtgggtcttt     300

ggcggtggca cgaaactgac cgttctgaaa                                      330


<210>  42
<211>  110
<212>  PRT
<213>  Artificial Sequence

<220>
<223>  Synthesized

<400>  42

Gln Ala Val Val Thr Gln Glu Pro Ser Leu Thr Val Ser Pro Gly Gly
1               5                   10                  15


Thr Val Thr Leu Thr Cys Arg Ser Ser Thr Gly Ala Val Thr Thr Ser
            20                  25                  30


Asn Tyr Ala Asn Trp Val Gln Gln Lys Pro Asp His Leu Phe Arg Gly
        35                  40                  45


Leu Ile Gly Gly Thr Asn Lys Arg Ala Pro Gly Thr Pro Ala Arg Phe
    50                  55                  60


Ser Gly Ser Leu Leu Gly Asp Lys Ala Ala Leu Thr Ile Ser Gly Ala
65                  70                  75                  80


Gln Pro Glu Asp Glu Ala Glu Tyr Tyr Cys Ala Leu Trp Tyr Ser Asn
                85                  90                  95


Leu Trp Val Phe Gly Gly Gly Thr Lys Leu Thr Val Leu Lys
            100                 105                 110


<210>  43
<211>  2088
<212>  DNA
<213>  Artificial Sequence

<220>
<223>  Synthesized

<400>  43
caagctgttg tgacccaaga accgagtctg accgtgtctc cgggcggcac cgttacgctg      60

acctgtcgct cctctaccgg cgcagtcacc acgagcaact atgcaaattg ggtgcagcaa 120

aaaccggatc atctgtttcg tggcctgatt ggcggtacga acaaacgtgc gccggggacc 180

ccggcacgtt tttcgggcag cctgctgggc gataaagcag cactgaccat cagtggtgcg 240

cagccggaag atgaagcaga atattactgc gctctgtggt atagcaacct gtgggtcttt 300

ggcggtggca cgaaactgac cgttctgaaa cgcacagtgg cagcccccag cgtcttcatt 360

tttccccctt ccgatgaaca gctgaagtcc ggcactgctt ctgtggtctg tctgctgaac 420

aatttctatc ccagagaggc caaggtgcag tggaaagtgg acaacgctct gcagtccggc 480

aacagccagg agagtgtgac cgaacaggat agtaaggaca gcacatattc tctgtctagt 540

accctgacac tgagtaaggc agattacgag aagcacaaag tgtatgcctg cgaagtcact 600

catcagggac tgtcaagccc cgtgaccaag agcttcaacc ggggcgagtg tgacaaaact 660

cacacatgcc caccgtgccc agcacctcca gtcgccggac cgtcagtctt cctcttccct 720

ccaaaaccca aggacaccct catgatctcc cggacccctg aggtcacatg cgtggtggtg 780

gacgtgagcc acgaagaccc tgaggtcaag ttcaactggt acgtggacgg cgtggaggtg 840

cataatgcca agacaaagcc gcgggaggag cagtacaaca gcacgtaccg tgtggtcagc 900

gtcctcaccg tcctgcacca ggactggctg aatggcaagg agtacaagtg caaggtctcc 960

aacaaaggcc tcccaagctc catcgagaaa accatctcca aagccaaagg gcagccccga 1020

gaaccacagg tgtgcaccct gcctccatcc cgggatgagc tgaccaagaa ccaggtcagc 1080

ctgtcctgcg cggtcaaagg cttctatccc agcgacatcg ccgtggagtg ggagagcaat 1140

gggcagccgg agaacaacta caagaccacg cctcccgtgc tggactccga cggctccttc 1200

ttcctcgtca gcaagctcac cgtggacaag agcaggtggc agcaggggaa cgtcttctca 1260

tgctccgtga tgcatgaggc tctgcacaac cactacacgc agaagagcct ctccctgtct 1320

ccgggtaaag gaggcagcgg gggaagcggc ggcggagggt ctggaggagg gggcagtgac 1380

atccagatga cccagagccc cagcagcctg agcaccagcg tgggcgaccg cgtgaccctg 1440

acctgcaagg ccagccagga cgtgggcacc gccgtggact ggtaccagca gaagcccggc 1500

cagagcccca agctgctgat ctactgggcc agcacccgcc acaccggcat ccccagccgc 1560

ttcagcggca gcggcagcgg caccgacttc accctgacca tcagcagcct gcagcccgag 1620

gacttcgccg actactactg ccagcagtac aacagctacc ccctgacctt cggccccggc 1680

accaaggtgg acatcaaggg tggcggtggc tcgggcggtg gtgggtcggg tggcggcgga 1740

tctgaggtgc agctggtgca gagcggcccc gaggtgaaga agcccggcgc caccgtgaag 1800

atcagctgca agaccagcgg ctacaccttc accgagtaca ccatccactg ggtgaagcag 1860

gcccccggca agggcctgga gtggatcggc aacatcaacc ccaacaacgg cggcaccacc 1920

tacaaccaga agttcgagga caaggccacc ctgaccgtgg acaagagcac cgacaccgcc 1980

tacatggagc tgagcagcct gcgcagcgag gacaccgccg tgtactactg cgccgccggc     2040

tggaacttcg actactgggg ccagggcacc ctacttacag taagtagc                 2088

<210>  44
<211>  696
<212>  PRT
<213>  Artificial Sequence

<220>
<223>  Synthesized

<400>  44

Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Asp His Leu Phe Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Asp Lys Ala Ala Leu Thr Ile Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu Lys Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys Asp Lys Thr His Thr Cys Pro
    210                 215                 220

Pro Cys Pro Ala Pro Pro Val Ala Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Gly Leu Pro Ser Ser Ile Glu Lys Thr Ile Ser Lys Ala Lys
                325                 330                 335

Gly Gln Pro Arg Glu Pro Gln Val Cys Thr Leu Pro Pro Ser Arg Asp
            340                 345                 350

Glu Leu Thr Lys Asn Gln Val Ser Leu Ser Cys Ala Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Val Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Lys Gly Gly Ser Gly Gly
        435                 440                 445

Ser Gly Gly Gly Gly Ser Gly Gly Gly Gly Ser Asp Ile Gln Met Thr
    450                 455                 460

Gln Ser Pro Ser Ser Leu Ser Thr Ser Val Gly Asp Arg Val Thr Leu
465                 470                 475                 480

Thr Cys Lys Ala Ser Gln Asp Val Gly Thr Ala Val Asp Trp Tyr Gln
                485                 490                 495

Gln Lys Pro Gly Gln Ser Pro Lys Leu Leu Ile Tyr Trp Ala Ser Thr
            500                 505                 510

Arg His Thr Gly Ile Pro Ser Arg Phe Ser Gly Ser Gly Ser Gly Thr
        515                 520                 525

Asp Phe Thr Leu Thr Ile Ser Ser Leu Gln Pro Glu Asp Phe Ala Asp
    530                 535                 540

Tyr Tyr Cys Gln Gln Tyr Asn Ser Tyr Pro Leu Thr Phe Gly Pro Gly
545                 550                 555                 560

Thr Lys Val Asp Ile Lys Gly Gly Gly Gly Ser Gly Gly Gly Gly Ser
                565                 570                 575

Gly Gly Gly Gly Ser Glu Val Gln Leu Val Gln Ser Gly Pro Glu Val
            580                 585                 590

Lys Lys Pro Gly Ala Thr Val Lys Ile Ser Cys Lys Thr Ser Gly Tyr
        595                 600                 605

Thr Phe Thr Glu Tyr Thr Ile His Trp Val Lys Gln Ala Pro Gly Lys
    610                 615                 620

Gly Leu Glu Trp Ile Gly Asn Ile Asn Pro Asn Asn Gly Gly Thr Thr
625                 630                 635                 640

Tyr Asn Gln Lys Phe Glu Asp Lys Ala Thr Leu Thr Val Asp Lys Ser
                645                 650                 655

Thr Asp Thr Ala Tyr Met Glu Leu Ser Ser Leu Arg Ser Glu Asp Thr
            660                 665                 670

Ala Val Tyr Tyr Cys Ala Ala Gly Trp Asn Phe Asp Tyr Trp Gly Gln
        675                 680                 685

Gly Thr Leu Leu Thr Val Ser Ser

690                 695

<210>  45
<211>  375
<212>  DNA
<213>  Artificial Sequence

<220>
<223>  Synthesized

<400>  45
gaggtgcagc tggtggagtc tgggggaggc ttggtccagc ctggggggtc cctgcgtctc      60

tcctgtgcag cctctggatt caccttcaat acctacgcca tgaactgggt ccgccaggct      120

ccgggaaagg gtttggaatg ggttgctcgc attcgcagta aatataataa ttatgcaaca      180

tattatgccg attcagtgaa agaccgtttc accatctccc gcgatgattc aaagaacacg      240

ctctatctgc aaatgaacag cctgcgcgct gaggacacgg ccgtgtatta ctgcgtgcgt      300

catggcaact ttggtaattc ttatgtgagc tggttcgcct actggggcca gggaaccctg      360

gtcaccgtca gctct                                                       375

<210>  46
<211>  125
<212>  PRT
<213>  Artificial Sequence

<220>
<223>  Synthesized

<400>  46

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210>  47
<211>  1329
<212>  DNA
<213>  Artificial Sequence

<220>
<223>  Synthesized

<400>  47
caagctgttg tgacccaaga accgagtctg accgtgtctc cgggcggcac cgttacgctg      60

acctgtcgct cctctaccgg cgcagtcacc acgagcaact atgcaaattg ggtgcagcaa     120

aaaccggatc atctgtttcg tggcctgatt ggcggtacga acaaacgtgc gccggggacc     180

ccggcacgtt tttcgggcag cctgctgggc gataaagcag cactgaccat cagtggtgcg     240

cagccggaag atgaagcaga atattactgc gctctgtggt atagcaacct gtgggtcttt     300

ggcggtggca cgaaactgac cgttctgaaa cgcacagtgg cagcccccag cgtcttcatt     360

tttccccctt ccgatgaaca gctgaagtcc ggcactgctt ctgtggtctg tctgctgaac     420

aatttctatc ccagagaggc caaggtgcag tggaaagtgg acaacgctct gcagtccggc     480

aacagccagg agagtgtgac cgaacaggat agtaaggaca gcacatattc tctgtctagt     540

accctgacac tgagtaaggc agattacgag aagcacaaag tgtatgcctg cgaagtcact     600

catcagggac tgtcaagccc cgtgaccaag agcttcaacc ggggcgagtg tgacaaaact     660

cacacatgcc caccgtgccc agcacctcca gtcgccggac cgtcagtctt cctcttccct     720

ccaaaaccca aggacaccct catgatctcc cggacccctg aggtcacatg cgtggtggtg     780

gacgtgagcc acgaagaccc tgaggtcaag ttcaactggt acgtggacgg cgtggaggtg     840

cataatgcca agacaaagcc gcgggaggag cagtacaaca gcacgtaccg tgtggtcagc     900

gtcctcaccg tcctgcacca ggactggctg aatggcaagg agtacaagtg caaggtctcc     960

aacaaaggcc tcccaagctc catcgagaaa accatctcca aagccaaagg gcagccccga    1020

gaaccacagg tgtgcaccct gcctccatcc cgggatgagc tgaccaagaa ccaggtcagc    1080

ctgtcctgcg cggtcaaagg cttctatccc agcgacatcg ccgtggagtg ggagagcaat    1140

gggcagccgg agaacaacta caagaccacg cctcccgtgc tggactccga cggctccttc    1200

ttcctcgtca gcaagctcac cgtggacaag agcaggtggc agcaggggaa cgtcttctca    1260

tgctccgtga tgcatgaggc tctgcacaac cactacacgc agaagagcct ctccctgtct    1320

ccgggtaaa                                                            1329

<210>  48

<211>  443
<212>  PRT
<213>  Artificial Sequence

<220>
<223>  Synthesized

<400>  48

Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Asp His Leu Phe Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Asp Lys Ala Ala Leu Thr Ile Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu Lys Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys Asp Lys Thr His Thr Cys Pro
    210                 215                 220

Pro Cys Pro Ala Pro Pro Val Ala Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Gly Leu Pro Ser Ser Ile Glu Lys Thr Ile Ser Lys Ala Lys
                325                 330                 335

Gly Gln Pro Arg Glu Pro Gln Val Cys Thr Leu Pro Pro Ser Arg Asp
            340                 345                 350

Glu Leu Thr Lys Asn Gln Val Ser Leu Ser Cys Ala Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Val Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Lys
        435                 440

<210>  49
<211>  1380
<212>  DNA
<213>  Artificial Sequence

<220>
<223>  Synthesized

<400>  49
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccagagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc     600

gcctacatgg agctgagcag cctgcgcagc gaggacaccg ccgtgtacta ctgcgccgcc     660

ggctggaact tcgactactg gggccagggc accctactta cagtaagtag caccacgacg     720

ccagcgccgc gaccaccaac accggcgccc accatcgcgt cgcagcccct gtccctgcgc     780

ccagaggcgt gccggccagc ggcggggggc gcagtgcaca cgagggggct ggacttcgcc     840

tgtgatatct acatctgggc gcccttggcc gggacttgtg gggtccttct cctgtcactg     900

gttatcaccc tttactgcaa acggggcaga aagaaactcc tgtatatatt caaacaacca     960

tttatgagac cagtacaaac tactcaagag gaagatggct gtagctgccg atttccagaa    1020

gaagaagaag gaggatgtga actgagagtg aagttcagca ggagcgcaga cgcccccgcg    1080

tacaagcagg gccagaacca gctctataac gagctcaatc taggacgaag agaggagtac    1140

gatgttttgg acaagagacg tggccgggac cctgagatgg ggggaaagcc gagaaggaag    1200

aaccctcagg aaggcctgta caatgaactg cagaaagata agatggcgga ggcctacagt    1260

gagattggga tgaaaggcga gcgccggagg ggcaaggggc acgatggcct ttaccagggt    1320

ctcagtacag ccaccaagga cacctacgac gcccttcaca tgcaggccct gccccctcgc    1380


<210>  50
<211>  460
<212>  PRT
<213>  Artificial Sequence

<220>
<223>  Synthesized

<400>  50

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30
Val Asp Trp Tyr Gln Gln Lys Pro Gly Gln Ser Pro Lys Leu Leu Ile
        35                  40                  45
Tyr Trp Ala Ser Thr Arg His Thr Gly Ile Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Asp Tyr Tyr Cys Gln Gln Tyr Asn Ser Tyr Pro Leu
                85                  90                  95
Thr Phe Gly Pro Gly Thr Lys Val Asp Ile Lys Gly Gly Gly Gly Ser
            100                 105                 110
Gly Gly Gly Gly Ser Gly Gly Gly Gly Ser Glu Val Gln Leu Val Gln
        115                 120                 125
Ser Gly Pro Glu Val Lys Lys Pro Gly Ala Thr Val Lys Ile Ser Cys
    130                 135                 140
Lys Thr Ser Gly Tyr Thr Phe Thr Glu Tyr Thr Ile His Trp Val Lys
145                 150                 155                 160
Gln Ala Pro Gly Lys Gly Leu Glu Trp Ile Gly Asn Ile Asn Pro Asn
                165                 170                 175
Asn Gly Gly Thr Thr Tyr Asn Gln Lys Phe Glu Asp Lys Ala Thr Leu
            180                 185                 190
Thr Val Asp Lys Ser Thr Asp Thr Ala Tyr Met Glu Leu Ser Ser Leu
        195                 200                 205
Arg Ser Glu Asp Thr Ala Val Tyr Tyr Cys Ala Ala Gly Trp Asn Phe
    210                 215                 220
Asp Tyr Trp Gly Gln Gly Thr Leu Leu Thr Val Ser Ser Thr Thr Thr
225                 230                 235                 240
Pro Ala Pro Arg Pro Pro Thr Pro Ala Pro Thr Ile Ala Ser Gln Pro
                245                 250                 255
Leu Ser Leu Arg Pro Glu Ala Cys Arg Pro Ala Ala Gly Gly Ala Val

260                 265                 270

His Thr Arg Gly Leu Asp Phe Ala Cys Asp Ile Tyr Ile Trp Ala Pro
        275                 280                 285

Leu Ala Gly Thr Cys Gly Val Leu Leu Leu Ser Leu Val Ile Thr Leu
    290                 295                 300

Tyr Cys Lys Arg Gly Arg Lys Lys Leu Leu Tyr Ile Phe Lys Gln Pro
305                 310                 315                 320

Phe Met Arg Pro Val Gln Thr Thr Gln Glu Glu Asp Gly Cys Ser Cys
                325                 330                 335

Arg Phe Pro Glu Glu Glu Glu Gly Gly Cys Glu Leu Arg Val Lys Phe
            340                 345                 350

Ser Arg Ser Ala Asp Ala Pro Ala Tyr Lys Gln Gly Gln Asn Gln Leu
        355                 360                 365

Tyr Asn Glu Leu Asn Leu Gly Arg Arg Glu Glu Tyr Asp Val Leu Asp
    370                 375                 380

Lys Arg Arg Gly Arg Asp Pro Glu Met Gly Gly Lys Pro Arg Arg Lys
385                 390                 395                 400

Asn Pro Gln Glu Gly Leu Tyr Asn Glu Leu Gln Lys Asp Lys Met Ala
                405                 410                 415

Glu Ala Tyr Ser Glu Ile Gly Met Lys Gly Glu Arg Arg Arg Gly Lys
            420                 425                 430

Gly His Asp Gly Leu Tyr Gln Gly Leu Ser Thr Ala Thr Lys Asp Thr
        435                 440                 445

Tyr Asp Ala Leu His Met Gln Ala Leu Pro Pro Arg
    450                 455                 460

<210>  51
<211>  321
<212>  DNA
<213>  Artificial Sequence

<220>
<223>  Synthesized

<400>  51
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggcaagagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa g 321

<210> 52
<211> 107
<212> PRT
<213> Artificial Sequence

<220>
<223> Synthesized

<400> 52

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Lys Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys
            100                 105

<210> 53
<211> 711
<212> DNA
<213> Artificial Sequence

<220>
<223> Synthesized

<400> 53
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggcaagagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag c 711

<210> 54
<211> 237
<212> PRT
<213> Artificial Sequence

<220>
<223> Synthesized

<400> 54

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Lys Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu
        195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210>  55
<211>  711
<212>  DNA
<213>  Artificial Sequence

<220>
<223>  Synthesized

<400>  55
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggcaagagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc     600

gcctacatgg agctgagcag cctgcgcagc gaggacaccg ccgtgtacta ctgcgccgcc     660

ggctggaact tcgactactg gggccagggc accctactta cagtaagtag c              711

<210>  56
<211>  237
<212>  PRT

<213>  Artificial Sequence

<220>
<223>  Synthesized

<400>  56

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Lys Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Gly Gly Gly Gly Ser
            100                 105                 110

Gly Gly Gly Gly Ser Gly Gly Gly Gly Ser Glu Val Gln Leu Val Gln
        115                 120                 125

Ser Gly Pro Glu Val Lys Lys Pro Gly Ala Thr Val Lys Ile Ser Cys
    130                 135                 140

Lys Thr Ser Gly Tyr Thr Phe Thr Glu Tyr Thr Ile His Trp Val Lys
145                 150                 155                 160

Gln Ala Pro Gly Lys Gly Leu Glu Trp Ile Gly Asn Ile Asn Pro Asn
                165                 170                 175

Asn Gly Gly Thr Thr Tyr Asn Gln Lys Phe Glu Asp Lys Ala Thr Leu
            180                 185                 190

Thr Val Asp Lys Ser Thr Asp Thr Ala Tyr Met Glu Leu Ser Ser Leu
        195                 200                 205

Arg Ser Glu Asp Thr Ala Val Tyr Tyr Cys Ala Ala Gly Trp Asn Phe
    210                 215                 220

Asp Tyr Trp Gly Gln Gly Thr Leu Leu Thr Val Ser Ser
225                 230                 235

<210>  57
<211>  1725
<212>  DNA
<213>  Artificial Sequence

<220>
<223>  Synthesized

<400>  57
gaggtgcagc tggtgcagag cggccccgag gtgaagaagc ccggcgccac cgtgaagatc      60

agctgcaaga ccagcggcta caccttcacc gagtacacca tccactgggt gaagcaggcc     120

cccggcaagg gcctggagtg gatcggcaac atcaacccca acaacggcgg caccacctac     180

aaccagaagt tcgaggacaa ggccaccctg accgtggaca agagcaccga caccgcctac     240

atggagctga gcagcctgcg cagcgaggac accgccgtgt actactgcgc cgccggctgg     300

aacttcgact actggggcca gggcacccta cttacagtaa gtagcgctag tacgaaaggt     360

ccggaggtgc agctggtgga gtctggggga ggcttggtcc agcctggggg gtccctgcgt     420

ctctcctgtg cagcctctgg attcaccttc aatacctacg ccatgaactg ggtccgccag     480

gctccgggaa agggtttgga atgggttgct cgcattcgca gtaaatataa taattatgca     540

acatattatg ccgattcagt gaaagaccgt ttcaccatct cccgcgatga ttcaaagaac     600

acgctctatc tgcaaatgaa cagcctgcgc gctgaggaca cggccgtgta ttactgcgtg     660

cgtcatggca actttggtaa ttcttatgtg agctggttcg cctactgggg ccagggaacc     720

ctggtcaccg tcagctctgc ctccaccaag ggcccatcgg tcttccccct ggcaccctcc     780

tccaagagca cctctggggg cacagcggcc ctgggctgcc tggtcaagga ctacttcccc     840

gaaccggtga cggtgtcgtg gaactcaggc gccctgacca gcggcgtgca caccttcccg     900

gctgtcctac agtcctcagg actctactcc ctcagcagcg tggtgactgt gccctctagc     960

agcttgggca cccagaccta catctgcaac gtgaatcaca agcccagcaa caccaaggtg    1020

gacaagaaag ttgaacccaa atcttgcgac aaaactcaca catgcccacc gtgcccagca    1080

cctccagtcg ccggaccgtc agtcttcctc ttccctccaa aacccaagga caccctcatg    1140

atctcccgga cccctgaggt cacatgcgtg gtggtggacg tgagccacga agaccctgag    1200

gtcaagttca actggtacgt ggacggcgtg gaggtgcata atgccaagac aaagccgcgg    1260

gaggagcagt acaacagcac gtaccgtgtg gtcagcgtcc tcaccgtcct gcaccaggac    1320

tggctgaatg gcaaggagta caagtgcaag gtctccaaca aaggcctccc aagctccatc    1380

gagaaaacca tctccaaagc caaagggcag ccccgagaac cacaggtgta caccctgcct    1440

ccatgccggg atgagctgac caagaaccag gtcagcctgt ggtgcctggt caaaggcttc    1500

tatcccagcg acatcgccgt ggagtgggag agcaatgggc agccggagaa caactacaag 1560

accacgcctc ccgtgctgga ctccgacggc tccttcttcc tctacagcaa gctcaccgtg 1620

gacaagagca ggtggcagca ggggaacgtc ttctcatgct ccgtgatgca tgaggctctg 1680

cacaaccact acacgcagaa gagcctctcc ctgtctccgg gtaaa 1725

<210> 58
<211> 575
<212> PRT
<213> Artificial Sequence

<220>
<223> Synthesized

<400> 58

Glu Val Gln Leu Val Gln Ser Gly Pro Glu Val Lys Lys Pro Gly Ala
1               5                   10                  15

Thr Val Lys Ile Ser Cys Lys Thr Ser Gly Tyr Thr Phe Thr Glu Tyr
            20                  25                  30

Thr Ile His Trp Val Lys Gln Ala Pro Gly Lys Gly Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser Ala Ser Thr Lys Gly Pro Glu Val Gln Leu Val Glu Ser
        115                 120                 125

Gly Gly Gly Leu Val Gln Pro Gly Gly Ser Leu Arg Leu Ser Cys Ala
    130                 135                 140

Ala Ser Gly Phe Thr Phe Asn Thr Tyr Ala Met Asn Trp Val Arg Gln
145                 150                 155                 160

Ala Pro Gly Lys Gly Leu Glu Trp Val Ala Arg Ile Arg Ser Lys Tyr
                165                 170                 175

Asn Asn Tyr Ala Thr Tyr Tyr Ala Asp Ser Val Lys Asp Arg Phe Thr
        180                 185                 190

Ile Ser Arg Asp Asp Ser Lys Asn Thr Leu Tyr Leu Gln Met Asn Ser
    195                 200                 205

Leu Arg Ala Glu Asp Thr Ala Val Tyr Tyr Cys Val Arg His Gly Asn
    210                 215                 220

Phe Gly Asn Ser Tyr Val Ser Trp Phe Ala Tyr Trp Gly Gln Gly Thr
225                 230                 235                 240

Leu Val Thr Val Ser Ser Ala Ser Thr Lys Gly Pro Ser Val Phe Pro
                245                 250                 255

Leu Ala Pro Ser Ser Lys Ser Thr Ser Gly Gly Thr Ala Ala Leu Gly
            260                 265                 270

Cys Leu Val Lys Asp Tyr Phe Pro Glu Pro Val Thr Val Ser Trp Asn
        275                 280                 285

Ser Gly Ala Leu Thr Ser Gly Val His Thr Phe Pro Ala Val Leu Gln
    290                 295                 300

Ser Ser Gly Leu Tyr Ser Leu Ser Ser Val Val Thr Val Pro Ser Ser
305                 310                 315                 320

Ser Leu Gly Thr Gln Thr Tyr Ile Cys Asn Val Asn His Lys Pro Ser
                325                 330                 335

Asn Thr Lys Val Asp Lys Lys Val Glu Pro Lys Ser Cys Asp Lys Thr
            340                 345                 350

His Thr Cys Pro Pro Cys Pro Ala Pro Pro Val Ala Gly Pro Ser Val
        355                 360                 365

Phe Leu Phe Pro Pro Lys Pro Lys Asp Thr Leu Met Ile Ser Arg Thr
    370                 375                 380

Pro Glu Val Thr Cys Val Val Val Asp Val Ser His Glu Asp Pro Glu
385                 390                 395                 400

Val Lys Phe Asn Trp Tyr Val Asp Gly Val Glu Val His Asn Ala Lys
                405                 410                 415

Thr Lys Pro Arg Glu Glu Gln Tyr Asn Ser Thr Tyr Arg Val Val Ser
            420                 425                 430

Val Leu Thr Val Leu His Gln Asp Trp Leu Asn Gly Lys Glu Tyr Lys
        435                 440                 445

Cys Lys Val Ser Asn Lys Gly Leu Pro Ser Ser Ile Glu Lys Thr Ile
    450                 455                 460

Ser Lys Ala Lys Gly Gln Pro Arg Glu Pro Gln Val Tyr Thr Leu Pro
465                 470                 475                 480

Pro Cys Arg Asp Glu Leu Thr Lys Asn Gln Val Ser Leu Trp Cys Leu
                485                 490                 495

Val Lys Gly Phe Tyr Pro Ser Asp Ile Ala Val Glu Trp Glu Ser Asn
            500                 505                 510

Gly Gln Pro Glu Asn Asn Tyr Lys Thr Thr Pro Pro Val Leu Asp Ser
        515                 520                 525

Asp Gly Ser Phe Phe Leu Tyr Ser Lys Leu Thr Val Asp Lys Ser Arg
    530                 535                 540

Trp Gln Gln Gly Asn Val Phe Ser Cys Ser Val Met His Glu Ala Leu
545                 550                 555                 560

His Asn His Tyr Thr Gln Lys Ser Leu Ser Leu Ser Pro Gly Lys
                565                 570                 575

<210>  59
<211>  1686
<212>  DNA
<213>  Artificial Sequence

<220>
<223>  Synthesized

<400>  59
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccccagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa gactgttgct gcaccgtcag tgttcatctt ccctccacaa     360

gctgttgtga cccaagaacc gagtctgacc gtgtctccgg gcggcaccgt tacgctgacc     420

tgtcgctcct ctaccggcgc agtcaccacg agcaactatg caaattgggt gcagcaaaaa     480

ccggatcatc tgtttcgtgg cctgattggc ggtacgaaca aacgtgcgcc ggggaccccg     540

gcacgttttt cgggcagcct gctgggcgat aaagcagcac tgaccatcag tggtgcgcag 600

ccggaagatg aagcagaata ttactgcgct ctgtggtata gcaacctgtg ggtctttggc 660

ggtggcacga aactgaccgt tctgaaacgc acagtggcag cccccagcgt cttcattttt 720

cccccttccg atgaacagct gaagtccggc actgcttctg tggtctgtct gctgaacaat 780

ttctatccca gagaggccaa ggtgcagtgg aaagtggaca acgctctgca gtccggcaac 840

agccaggaga gtgtgaccga acaggatagt aaggacagca catattctct gtctagtacc 900

ctgacactga gtaaggcaga ttacgagaag cacaaagtgt atgcctgcga agtcactcat 960

cagggactgt caagccccgt gaccaagagc ttcaaccggg gcgagtgtga caaaactcac 1020

acatgcccac cgtgcccagc acctccagtc gccggaccgt cagtcttcct cttccctcca 1080

aaacccaagg acaccctcat gatctcccgg acccctgagg tcacatgcgt ggtggtggac 1140

gtgagccacg aagaccctga ggtcaagttc aactggtacg tggacggcgt ggaggtgcat 1200

aatgccaaga caaagccgcg ggaggagcag tacaacagca cgtaccgtgt ggtcagcgtc 1260

ctcaccgtcc tgcaccagga ctggctgaat ggcaaggagt acaagtgcaa ggtctccaac 1320

aaaggcctcc caagctccat cgagaaaacc atctccaaag ccaaagggca gccccgagaa 1380

ccacaggtgt gcaccctgcc tccatcccgg gatgagctga ccaagaacca ggtcagcctg 1440

tcctgcgcgg tcaaaggctt ctatcccagc gacatcgccg tggagtggga gagcaatggg 1500

cagccggaga acaactacaa gaccacgcct cccgtgctgg actccgacgg ctccttcttc 1560

ctcgtcagca agctcaccgt ggacaagagc aggtggcagc aggggaacgt cttctcatgc 1620

tccgtgatgc atgaggctct gcacaaccac tacacgcaga agagcctctc cctgtctccg 1680

ggtaaa 1686

<210> 60
<211> 562
<212> PRT
<213> Artificial Sequence

<220>
<223> Synthesized

<400> 60

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Pro Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Thr Val Ala Ala Pro
            100                 105                 110

Ser Val Phe Ile Phe Pro Pro Gln Ala Val Val Thr Gln Glu Pro Ser
        115                 120                 125

Leu Thr Val Ser Pro Gly Gly Thr Val Thr Leu Thr Cys Arg Ser Ser
    130                 135                 140

Thr Gly Ala Val Thr Thr Ser Asn Tyr Ala Asn Trp Val Gln Gln Lys
145                 150                 155                 160

Pro Asp His Leu Phe Arg Gly Leu Ile Gly Gly Thr Asn Lys Arg Ala
                165                 170                 175

Pro Gly Thr Pro Ala Arg Phe Ser Gly Ser Leu Leu Gly Asp Lys Ala
            180                 185                 190

Ala Leu Thr Ile Ser Gly Ala Gln Pro Glu Asp Glu Ala Glu Tyr Tyr
        195                 200                 205

Cys Ala Leu Trp Tyr Ser Asn Leu Trp Val Phe Gly Gly Gly Thr Lys
    210                 215                 220

Leu Thr Val Leu Lys Arg Thr Val Ala Ala Pro Ser Val Phe Ile Phe
225                 230                 235                 240

Pro Pro Ser Asp Glu Gln Leu Lys Ser Gly Thr Ala Ser Val Val Cys
                245                 250                 255

Leu Leu Asn Asn Phe Tyr Pro Arg Glu Ala Lys Val Gln Trp Lys Val
            260                 265                 270

Asp Asn Ala Leu Gln Ser Gly Asn Ser Gln Glu Ser Val Thr Glu Gln
        275                 280                 285

Asp Ser Lys Asp Ser Thr Tyr Ser Leu Ser Ser Thr Leu Thr Leu Ser
    290                 295                 300

Lys Ala Asp Tyr Glu Lys His Lys Val Tyr Ala Cys Glu Val Thr His
305                 310                 315                 320

Gln Gly Leu Ser Ser Pro Val Thr Lys Ser Phe Asn Arg Gly Glu Cys
                325                 330                 335

Asp Lys Thr His Thr Cys Pro Pro Cys Pro Ala Pro Pro Val Ala Gly
            340                 345                 350

Pro Ser Val Phe Leu Phe Pro Pro Lys Pro Lys Asp Thr Leu Met Ile
        355                 360                 365

Ser Arg Thr Pro Glu Val Thr Cys Val Val Val Asp Val Ser His Glu
    370                 375                 380

Asp Pro Glu Val Lys Phe Asn Trp Tyr Val Asp Gly Val Glu Val His
385                 390                 395                 400

Asn Ala Lys Thr Lys Pro Arg Glu Glu Gln Tyr Asn Ser Thr Tyr Arg
                405                 410                 415

Val Val Ser Val Leu Thr Val Leu His Gln Asp Trp Leu Asn Gly Lys
            420                 425                 430

Glu Tyr Lys Cys Lys Val Ser Asn Lys Gly Leu Pro Ser Ser Ile Glu
        435                 440                 445

Lys Thr Ile Ser Lys Ala Lys Gly Gln Pro Arg Glu Pro Gln Val Cys
    450                 455                 460

Thr Leu Pro Pro Ser Arg Asp Glu Leu Thr Lys Asn Gln Val Ser Leu
465                 470                 475                 480

Ser Cys Ala Val Lys Gly Phe Tyr Pro Ser Asp Ile Ala Val Glu Trp
                485                 490                 495

Glu Ser Asn Gly Gln Pro Glu Asn Asn Tyr Lys Thr Thr Pro Pro Val
            500                 505                 510

Leu Asp Ser Asp Gly Ser Phe Phe Leu Val Ser Lys Leu Thr Val Asp
        515                 520                 525

Lys Ser Arg Trp Gln Gln Gly Asn Val Phe Ser Cys Ser Val Met His
    530                 535                 540

Glu Ala Leu His Asn His Tyr Thr Gln Lys Ser Leu Ser Leu Ser Pro

545                 550                 555                 560

Gly Lys

<210>  61
<211>  1719
<212>  DNA
<213>  Artificial Sequence

<220>
<223>  Synthesized

<400>  61
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccccagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa gactgttgct gcaccgtcag tgttcatctt ccctccagag     360

gtgcagctgg tggagtctgg gggaggcttg gtccagcctg gggggtccct gcgtctctcc     420

tgtgcagcct ctggattcac cttcaatacc tacgccatga actgggtccg ccaggctccg     480

ggaaagggtt tggaatgggt tgctcgcatt cgcagtaaat ataataatta tgcaacatat     540

tatgccgatt cagtgaaaga ccgtttcacc atctcccgcg atgattcaaa gaacacgctc     600

tatctgcaaa tgaacagcct gcgcgctgag gacacggccg tgtattactg cgtgcgtcat     660

ggcaactttg gtaattctta tgtgagctgg ttcgcctact ggggccaggg aaccctggtc     720

accgtcagct ctgcctccac caagggccca tcggtcttcc ccctggcacc ctcctccaag     780

agcacctctg ggggcacagc ggccctgggc tgcctggtca aggactactt ccccgaaccg     840

gtgacggtgt cgtggaactc aggcgccctg accagcggcg tgcacacctt cccggctgtc     900

ctacagtcct caggactcta ctccctcagc agcgtggtga ctgtgccctc tagcagcttg     960

ggcacccaga cctacatctg caacgtgaat cacaagccca gcaacaccaa ggtggacaag    1020

aaagttgaac ccaaatcttg cgacaaaact cacacatgcc caccgtgccc agcacctcca    1080

gtcgccggac cgtcagtctt cctcttccct ccaaaaccca aggacaccct catgatctcc    1140

cggacccctg aggtcacatg cgtggtggtg gacgtgagcc acgaagaccc tgaggtcaag    1200

ttcaactggt acgtggacgg cgtggaggtg cataatgcca agacaaagcc gcgggaggag    1260

cagtacaaca gcacgtaccg tgtggtcagc gtcctcaccg tcctgcacca ggactggctg    1320

aatggcaagg agtacaagtg caaggtctcc aacaaaggcc tcccaagctc catcgagaaa    1380

accatctcca aagccaaagg gcagccccga gaaccacagg tgtacaccct gcctccatgc    1440

cgggatgagc tgaccaagaa ccaggtcagc ctgtggtgcc tggtcaaagg cttctatccc 1500

agcgacatcg ccgtggagtg ggagagcaat gggcagccgg agaacaacta caagaccacg 1560

cctcccgtgc tggactccga cggctccttc ttcctctaca gcaagctcac cgtggacaag 1620

agcaggtggc agcaggggaa cgtcttctca tgctccgtga tgcatgaggc tctgcacaac 1680

cactacacgc agaagagcct ctccctgtct ccgggtaaa 1719

<210> 62
<211> 573
<212> PRT
<213> Artificial Sequence

<220>
<223> Synthesized

<400> 62

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Pro Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Thr Val Ala Ala Pro
            100                 105                 110

Ser Val Phe Ile Phe Pro Pro Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Thr Phe Asn Thr Tyr Ala Met Asn Trp Val Arg Gln Ala Pro
145                 150                 155                 160

Gly Lys Gly Leu Glu Trp Val Ala Arg Ile Arg Ser Lys Tyr Asn Asn

                165                 170                 175

Tyr Ala Thr Tyr Tyr Ala Asp Ser Val Lys Asp Arg Phe Thr Ile Ser
            180                 185                 190

Arg Asp Asp Ser Lys Asn Thr Leu Tyr Leu Gln Met Asn Ser Leu Arg
        195                 200                 205

Ala Glu Asp Thr Ala Val Tyr Tyr Cys Val Arg His Gly Asn Phe Gly
    210                 215                 220

Asn Ser Tyr Val Ser Trp Phe Ala Tyr Trp Gly Gln Gly Thr Leu Val
225                 230                 235                 240

Thr Val Ser Ser Ala Ser Thr Lys Gly Pro Ser Val Phe Pro Leu Ala
                245                 250                 255

Pro Ser Ser Lys Ser Thr Ser Gly Gly Thr Ala Ala Leu Gly Cys Leu
            260                 265                 270

Val Lys Asp Tyr Phe Pro Glu Pro Val Thr Val Ser Trp Asn Ser Gly
        275                 280                 285

Ala Leu Thr Ser Gly Val His Thr Phe Pro Ala Val Leu Gln Ser Ser
    290                 295                 300

Gly Leu Tyr Ser Leu Ser Ser Val Val Thr Val Pro Ser Ser Ser Leu
305                 310                 315                 320

Gly Thr Gln Thr Tyr Ile Cys Asn Val Asn His Lys Pro Ser Asn Thr
                325                 330                 335

Lys Val Asp Lys Lys Val Glu Pro Lys Ser Cys Asp Lys Thr His Thr
            340                 345                 350

Cys Pro Pro Cys Pro Ala Pro Pro Val Ala Gly Pro Ser Val Phe Leu
        355                 360                 365

Phe Pro Pro Lys Pro Lys Asp Thr Leu Met Ile Ser Arg Thr Pro Glu
    370                 375                 380

Val Thr Cys Val Val Val Asp Val Ser His Glu Asp Pro Glu Val Lys
385                 390                 395                 400

Phe Asn Trp Tyr Val Asp Gly Val Glu Val His Asn Ala Lys Thr Lys
                405                 410                 415

Pro Arg Glu Glu Gln Tyr Asn Ser Thr Tyr Arg Val Val Ser Val Leu
            420                 425                 430

Thr Val Leu His Gln Asp Trp Leu Asn Gly Lys Glu Tyr Lys Cys Lys
        435                 440                 445

Val Ser Asn Lys Gly Leu Pro Ser Ser Ile Glu Lys Thr Ile Ser Lys
    450                 455                 460

Ala Lys Gly Gln Pro Arg Glu Pro Gln Val Tyr Thr Leu Pro Pro Cys
465                 470                 475                 480

Arg Asp Glu Leu Thr Lys Asn Gln Val Ser Leu Trp Cys Leu Val Lys
                485                 490                 495

Gly Phe Tyr Pro Ser Asp Ile Ala Val Glu Trp Glu Ser Asn Gly Gln
            500                 505                 510

Pro Glu Asn Asn Tyr Lys Thr Thr Pro Pro Val Leu Asp Ser Asp Gly
        515                 520                 525

Ser Phe Phe Leu Tyr Ser Lys Leu Thr Val Asp Lys Ser Arg Trp Gln
    530                 535                 540

Gln Gly Asn Val Phe Ser Cys Ser Val Met His Glu Ala Leu His Asn
545                 550                 555                 560

His Tyr Thr Gln Lys Ser Leu Ser Leu Ser Pro Gly Lys
                565                 570

<210>  63
<211>  1692
<212>  DNA
<213>  Artificial Sequence

<220>
<223>  Synthesized

<400>  63
gaggtgcagc tggtgcagag cggccccgag gtgaagaagc ccggcgccac cgtgaagatc      60

agctgcaaga ccagcggcta caccttcacc gagtacacca tccactgggt gaagcaggcc     120

cccggcaagg gcctggagtg gatcggcaac atcaacccca acaacggcgg caccacctac     180

aaccagaagt tcgaggacaa ggccaccctg accgtggaca agagcaccga caccgcctac     240

atggagctga gcagcctgcg cagcgaggac accgccgtgt actactgcgc cgccggctgg     300

aacttcgact actggggcca gggcacccta cttacagtaa gtagcgctag tacgaaaggt     360

ccgcaagctg ttgtgaccca agaaccgagt ctgaccgtgt ctccgggcgg caccgttacg     420

ctgacctgtc gctcctctac cggcgcagtc accacgagca actatgcaaa ttgggtgcag 480

caaaaaccgg atcatctgtt tcgtggcctg attggcggta cgaacaaacg tgcgccgggg 540

accccggcac gtttttcggg cagcctgctg ggcgataaag cagcactgac catcagtggt 600

gcgcagccgg aagatgaagc agaatattac tgcgctctgt ggtatagcaa cctgtgggtc 660

tttggcggtg gcacgaaact gaccgttctg aaacgcacag tggcagcccc cagcgtcttc 720

atttttcccc cttccgatga acagctgaag tccggcactg cttctgtggt ctgtctgctg 780

aacaatttct atcccagaga ggccaaggtg cagtggaaag tggacaacgc tctgcagtcc 840

ggcaacagcc aggagagtgt gaccgaacag gatagtaagg acagcacata ttctctgtct 900

agtaccctga cactgagtaa ggcagattac gagaagcaca aagtgtatgc ctgcgaagtc 960

actcatcagg gactgtcaag ccccgtgacc aagagcttca accggggcga gtgtgacaaa 1020

actcacacat gcccaccgtg cccagcacct ccagtcgccg gaccgtcagt cttcctcttc 1080

cctccaaaac ccaaggacac cctcatgatc tcccggaccc ctgaggtcac atgcgtggtg 1140

gtggacgtga gccacgaaga ccctgaggtc aagttcaact ggtacgtgga cggcgtggag 1200

gtgcataatg ccaagacaaa gccgcgggag gagcagtaca acagcacgta ccgtgtggtc 1260

agcgtcctca ccgtcctgca ccaggactgg ctgaatggca aggagtacaa gtgcaaggtc 1320

tccaacaaag gcctcccaag ctccatcgag aaaaccatct ccaaagccaa agggcagccc 1380

cgagaaccac aggtgtgcac cctgcctcca tcccgggatg agctgaccaa gaaccaggtc 1440

agcctgtcct gcgcggtcaa aggcttctat cccagcgaca tcgccgtgga gtgggagagc 1500

aatgggcagc cggagaacaa ctacaagacc acgcctcccg tgctggactc cgacggctcc 1560

ttcttcctcg tcagcaagct caccgtggac aagagcaggt ggcagcaggg gaacgtcttc 1620

tcatgctccg tgatgcatga ggctctgcac aaccactaca cgcagaagag cctctccctg 1680

tctccgggta aa 1692

<210> 64
<211> 564
<212> PRT
<213> Artificial Sequence

<220>
<223> Synthesized

<400> 64

Glu Val Gln Leu Val Gln Ser Gly Pro Glu Val Lys Lys Pro Gly Ala
1               5                   10                  15

Thr Val Lys Ile Ser Cys Lys Thr Ser Gly Tyr Thr Phe Thr Glu Tyr
            20                  25                  30

Thr Ile His Trp Val Lys Gln Ala Pro Gly Lys Gly Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser Ala Ser Thr Lys Gly Pro Gln Ala Val Val Thr Gln Glu
        115                 120                 125

Pro Ser Leu Thr Val Ser Pro Gly Gly Thr Val Thr Leu Thr Cys Arg
    130                 135                 140

Ser Ser Thr Gly Ala Val Thr Thr Ser Asn Tyr Ala Asn Trp Val Gln
145                 150                 155                 160

Gln Lys Pro Asp His Leu Phe Arg Gly Leu Ile Gly Gly Thr Asn Lys
                165                 170                 175

Arg Ala Pro Gly Thr Pro Ala Arg Phe Ser Gly Ser Leu Leu Gly Asp
            180                 185                 190

Lys Ala Ala Leu Thr Ile Ser Gly Ala Gln Pro Glu Asp Glu Ala Glu
        195                 200                 205

Tyr Tyr Cys Ala Leu Trp Tyr Ser Asn Leu Trp Val Phe Gly Gly Gly
    210                 215                 220

Thr Lys Leu Thr Val Leu Lys Arg Thr Val Ala Ala Pro Ser Val Phe
225                 230                 235                 240

Ile Phe Pro Pro Ser Asp Glu Gln Leu Lys Ser Gly Thr Ala Ser Val
                245                 250                 255

Val Cys Leu Leu Asn Asn Phe Tyr Pro Arg Glu Ala Lys Val Gln Trp
            260                 265                 270

Lys Val Asp Asn Ala Leu Gln Ser Gly Asn Ser Gln Glu Ser Val Thr
        275                 280                 285

Glu Gln Asp Ser Lys Asp Ser Thr Tyr Ser Leu Ser Ser Thr Leu Thr
    290                 295                 300

Leu Ser Lys Ala Asp Tyr Glu Lys His Lys Val Tyr Ala Cys Glu Val
305                 310                 315                 320

Thr His Gln Gly Leu Ser Ser Pro Val Thr Lys Ser Phe Asn Arg Gly
                325                 330                 335

Glu Cys Asp Lys Thr His Thr Cys Pro Pro Cys Pro Ala Pro Pro Val
            340                 345                 350

Ala Gly Pro Ser Val Phe Leu Phe Pro Pro Lys Pro Lys Asp Thr Leu
        355                 360                 365

Met Ile Ser Arg Thr Pro Glu Val Thr Cys Val Val Val Asp Val Ser
    370                 375                 380

His Glu Asp Pro Glu Val Lys Phe Asn Trp Tyr Val Asp Gly Val Glu
385                 390                 395                 400

Val His Asn Ala Lys Thr Lys Pro Arg Glu Glu Gln Tyr Asn Ser Thr
                405                 410                 415

Tyr Arg Val Val Ser Val Leu Thr Val Leu His Gln Asp Trp Leu Asn
            420                 425                 430

Gly Lys Glu Tyr Lys Cys Lys Val Ser Asn Lys Gly Leu Pro Ser Ser
        435                 440                 445

Ile Glu Lys Thr Ile Ser Lys Ala Lys Gly Gln Pro Arg Glu Pro Gln
    450                 455                 460

Val Cys Thr Leu Pro Pro Ser Arg Asp Glu Leu Thr Lys Asn Gln Val
465                 470                 475                 480

Ser Leu Ser Cys Ala Val Lys Gly Phe Tyr Pro Ser Asp Ile Ala Val
                485                 490                 495

Glu Trp Glu Ser Asn Gly Gln Pro Glu Asn Asn Tyr Lys Thr Thr Pro
            500                 505                 510

Pro Val Leu Asp Ser Asp Gly Ser Phe Phe Leu Val Ser Lys Leu Thr
        515                 520                 525

Val Asp Lys Ser Arg Trp Gln Gln Gly Asn Val Phe Ser Cys Ser Val
    530                 535                 540

Met His Glu Ala Leu His Asn His Tyr Thr Gln Lys Ser Leu Ser Leu
545                 550                 555                 560

Ser Pro Gly Lys

<210>  65
<211>  1686
<212>  DNA
<213>  Artificial Sequence

<220>
<223>  Synthesized

<400>  65
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccagagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa gactgttgct gcaccgtcag tgttcatctt ccctccacaa     360

gctgttgtga cccaagaacc gagtctgacc gtgtctccgg gcggcaccgt tacgctgacc     420

tgtcgctcct ctaccggcgc agtcaccacg agcaactatg caaattgggt gcagcaaaaa     480

ccggatcatc tgtttcgtgg cctgattggc ggtacgaaca aacgtgcgcc ggggaccccg     540

gcacgttttt cgggcagcct gctgggcgat aaagcagcac tgaccatcag tggtgcgcag     600

ccggaagatg aagcagaata ttactgcgct ctgtggtata gcaacctgtg ggtctttggc     660

ggtggcacga aactgaccgt tctgaaacgc acagtggcag cccccagcgt cttcattttt     720

cccccttccg atgaacagct gaagtccggc actgcttctg tggtctgtct gctgaacaat     780

ttctatccca gagaggccaa ggtgcagtgg aaagtggaca acgctctgca gtccggcaac     840

agccaggaga gtgtgaccga acaggatagt aaggacagca catattctct gtctagtacc     900

ctgacactga gtaaggcaga ttacgagaag cacaaagtgt atgcctgcga agtcactcat     960

cagggactgt caagccccgt gaccaagagc ttcaaccggg gcgagtgtga caaaactcac    1020

acatgcccac cgtgcccagc acctccagtc gccggaccgt cagtcttcct cttccctcca    1080

aaacccaagg acaccctcat gatctcccgg acccctgagg tcacatgcgt ggtggtggac    1140

gtgagccacg aagaccctga ggtcaagttc aactggtacg tggacggcgt ggaggtgcat    1200

aatgccaaga caaagccgcg ggaggagcag tacaacagca cgtaccgtgt ggtcagcgtc    1260

ctcaccgtcc tgcaccagga ctggctgaat ggcaaggagt acaagtgcaa ggtctccaac    1320

aaaggcctcc caagctccat cgagaaaacc atctccaaag ccaaagggca gccccgagaa 1380

ccacaggtgt gcaccctgcc tccatcccgg gatgagctga ccaagaacca ggtcagcctg 1440

tcctgcgcgg tcaaaggctt ctatcccagc gacatcgccg tggagtggga gagcaatggg 1500

cagccggaga acaactacaa gaccacgcct cccgtgctgg actccgacgg ctccttcttc 1560

ctcgtcagca agctcaccgt ggacaagagc aggtggcagc aggggaacgt cttctcatgc 1620

tccgtgatgc atgaggctct gcacaaccac tacacgcaga agagcctctc cctgtctccg 1680

ggtaaa 1686

<210> 66
<211> 562
<212> PRT
<213> Artificial Sequence

<220>
<223> Synthesized

<400> 66

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Gln Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Thr Val Ala Ala Pro
            100                 105                 110

Ser Val Phe Ile Phe Pro Pro Gln Ala Val Val Thr Gln Glu Pro Ser
        115                 120                 125

Leu Thr Val Ser Pro Gly Gly Thr Val Thr Leu Thr Cys Arg Ser Ser
    130                 135                 140

Thr Gly Ala Val Thr Thr Ser Asn Tyr Ala Asn Trp Val Gln Gln Lys
145                 150                 155                 160

Pro Asp His Leu Phe Arg Gly Leu Ile Gly Gly Thr Asn Lys Arg Ala
                165                 170                 175

Pro Gly Thr Pro Ala Arg Phe Ser Gly Ser Leu Leu Gly Asp Lys Ala
            180                 185                 190

Ala Leu Thr Ile Ser Gly Ala Gln Pro Glu Asp Glu Ala Glu Tyr Tyr
        195                 200                 205

Cys Ala Leu Trp Tyr Ser Asn Leu Trp Val Phe Gly Gly Gly Thr Lys
    210                 215                 220

Leu Thr Val Leu Lys Arg Thr Val Ala Ala Pro Ser Val Phe Ile Phe
225                 230                 235                 240

Pro Pro Ser Asp Glu Gln Leu Lys Ser Gly Thr Ala Ser Val Val Cys
                245                 250                 255

Leu Leu Asn Asn Phe Tyr Pro Arg Glu Ala Lys Val Gln Trp Lys Val
            260                 265                 270

Asp Asn Ala Leu Gln Ser Gly Asn Ser Gln Glu Ser Val Thr Glu Gln
        275                 280                 285

Asp Ser Lys Asp Ser Thr Tyr Ser Leu Ser Ser Thr Leu Thr Leu Ser
    290                 295                 300

Lys Ala Asp Tyr Glu Lys His Lys Val Tyr Ala Cys Glu Val Thr His
305                 310                 315                 320

Gln Gly Leu Ser Ser Pro Val Thr Lys Ser Phe Asn Arg Gly Glu Cys
                325                 330                 335

Asp Lys Thr His Thr Cys Pro Pro Cys Pro Ala Pro Pro Val Ala Gly
            340                 345                 350

Pro Ser Val Phe Leu Phe Pro Pro Lys Pro Lys Asp Thr Leu Met Ile
        355                 360                 365

Ser Arg Thr Pro Glu Val Thr Cys Val Val Val Asp Val Ser His Glu
    370                 375                 380

Asp Pro Glu Val Lys Phe Asn Trp Tyr Val Asp Gly Val Glu Val His
385                 390                 395                 400

Asn Ala Lys Thr Lys Pro Arg Glu Glu Gln Tyr Asn Ser Thr Tyr Arg

405                 410                 415

Val Val Ser Val Leu Thr Val Leu His Gln Asp Trp Leu Asn Gly Lys
            420                 425                 430

Glu Tyr Lys Cys Lys Val Ser Asn Lys Gly Leu Pro Ser Ser Ile Glu
        435                 440                 445

Lys Thr Ile Ser Lys Ala Lys Gly Gln Pro Arg Glu Pro Gln Val Cys
    450                 455                 460

Thr Leu Pro Pro Ser Arg Asp Glu Leu Thr Lys Asn Gln Val Ser Leu
465                 470                 475                 480

Ser Cys Ala Val Lys Gly Phe Tyr Pro Ser Asp Ile Ala Val Glu Trp
                485                 490                 495

Glu Ser Asn Gly Gln Pro Glu Asn Asn Tyr Lys Thr Thr Pro Pro Val
            500                 505                 510

Leu Asp Ser Asp Gly Ser Phe Phe Leu Val Ser Lys Leu Thr Val Asp
        515                 520                 525

Lys Ser Arg Trp Gln Gln Gly Asn Val Phe Ser Cys Ser Val Met His
    530                 535                 540

Glu Ala Leu His Asn His Tyr Thr Gln Lys Ser Leu Ser Leu Ser Pro
545                 550                 555                 560

Gly Lys

<210>  67
<211>  1719
<212>  DNA
<213>  Artificial Sequence

<220>
<223>  Synthesized

<400>  67
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccagagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa gactgttgct gcaccgtcag tgttcatctt ccctccagag     360

gtgcagctgg tggagtctgg gggaggcttg gtccagcctg gggggtccct gcgtctctcc 420

tgtgcagcct ctggattcac cttcaatacc tacgccatga actgggtccg ccaggctccg 480

ggaaagggtt tggaatgggt tgctcgcatt cgcagtaaat ataataatta tgcaacatat 540

tatgccgatt cagtgaaaga ccgtttcacc atctcccgcg atgattcaaa gaacacgctc 600

tatctgcaaa tgaacagcct gcgcgctgag gacacggccg tgtattactg cgtgcgtcat 660

ggcaactttg gtaattctta tgtgagctgg ttcgcctact ggggccaggg aaccctggtc 720

accgtcagct ctgcctccac caagggccca tcggtcttcc ccctggcacc ctcctccaag 780

agcacctctg ggggcacagc ggccctgggc tgcctggtca aggactactt ccccgaaccg 840

gtgacggtgt cgtggaactc aggcgccctg accagcggcg tgcacacctt cccggctgtc 900

ctacagtcct caggactcta ctccctcagc agcgtggtga ctgtgccctc tagcagcttg 960

ggcacccaga cctacatctg caacgtgaat cacaagccca gcaacaccaa ggtggacaag 1020

aaagttgaac ccaaatcttg cgacaaaact cacacatgcc caccgtgccc agcacctcca 1080

gtcgccggac cgtcagtctt cctcttccct ccaaaaccca aggacaccct catgatctcc 1140

cggacccctg aggtcacatg cgtggtggtg gacgtgagcc acgaagaccc tgaggtcaag 1200

ttcaactggt acgtggacgg cgtggaggtg cataatgcca agacaaagcc gcgggaggag 1260

cagtacaaca gcacgtaccg tgtggtcagc gtcctcaccg tcctgcacca ggactggctg 1320

aatggcaagg agtacaagtg caaggtctcc aacaaaggcc tcccaagctc catcgagaaa 1380

accatctcca aagccaaagg gcagccccga gaaccacagg tgtacaccct gcctccatgc 1440

cgggatgagc tgaccaagaa ccaggtcagc ctgtggtgcc tggtcaaagg cttctatccc 1500

agcgacatcg ccgtggagtg ggagagcaat gggcagccgg agaacaacta caagaccacg 1560

cctcccgtgc tggactccga cggctccttc ttcctctaca gcaagctcac cgtggacaag 1620

agcaggtggc agcaggggaa cgtcttctca tgctccgtga tgcatgaggc tctgcacaac 1680

cactacacgc agaagagcct ctccctgtct ccgggtaaa 1719

<210> 68
<211> 573
<212> PRT
<213> Artificial Sequence

<220>
<223> Synthesized

<400> 68

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala

20                  25                  30

Val Asp Trp Tyr Gln Gln Lys Pro Gly Gln Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys Thr Val Ala Ala Pro
            100                 105                 110

Ser Val Phe Ile Phe Pro Pro Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Thr Phe Asn Thr Tyr Ala Met Asn Trp Val Arg Gln Ala Pro
145                 150                 155                 160

Gly Lys Gly Leu Glu Trp Val Ala Arg Ile Arg Ser Lys Tyr Asn Asn
                165                 170                 175

Tyr Ala Thr Tyr Tyr Ala Asp Ser Val Lys Asp Arg Phe Thr Ile Ser
            180                 185                 190

Arg Asp Asp Ser Lys Asn Thr Leu Tyr Leu Gln Met Asn Ser Leu Arg
        195                 200                 205

Ala Glu Asp Thr Ala Val Tyr Tyr Cys Val Arg His Gly Asn Phe Gly
    210                 215                 220

Asn Ser Tyr Val Ser Trp Phe Ala Tyr Trp Gly Gln Gly Thr Leu Val
225                 230                 235                 240

Thr Val Ser Ser Ala Ser Thr Lys Gly Pro Ser Val Phe Pro Leu Ala
                245                 250                 255

Pro Ser Ser Lys Ser Thr Ser Gly Gly Thr Ala Ala Leu Gly Cys Leu
            260                 265                 270

Val Lys Asp Tyr Phe Pro Glu Pro Val Thr Val Ser Trp Asn Ser Gly
        275                 280                 285

Ala Leu Thr Ser Gly Val His Thr Phe Pro Ala Val Leu Gln Ser Ser
    290                 295                 300

Gly Leu Tyr Ser Leu Ser Ser Val Val Thr Val Pro Ser Ser Ser Leu
305                 310                 315                 320

Gly Thr Gln Thr Tyr Ile Cys Asn Val Asn His Lys Pro Ser Asn Thr
                325                 330                 335

Lys Val Asp Lys Lys Val Glu Pro Lys Ser Cys Asp Lys Thr His Thr
            340                 345                 350

Cys Pro Pro Cys Pro Ala Pro Pro Val Ala Gly Pro Ser Val Phe Leu
        355                 360                 365

Phe Pro Pro Lys Pro Lys Asp Thr Leu Met Ile Ser Arg Thr Pro Glu
    370                 375                 380

Val Thr Cys Val Val Val Asp Val Ser His Glu Asp Pro Glu Val Lys
385                 390                 395                 400

Phe Asn Trp Tyr Val Asp Gly Val Glu Val His Asn Ala Lys Thr Lys
                405                 410                 415

Pro Arg Glu Glu Gln Tyr Asn Ser Thr Tyr Arg Val Val Ser Val Leu
            420                 425                 430

Thr Val Leu His Gln Asp Trp Leu Asn Gly Lys Glu Tyr Lys Cys Lys
        435                 440                 445

Val Ser Asn Lys Gly Leu Pro Ser Ser Ile Glu Lys Thr Ile Ser Lys
    450                 455                 460

Ala Lys Gly Gln Pro Arg Glu Pro Gln Val Tyr Thr Leu Pro Pro Cys
465                 470                 475                 480

Arg Asp Glu Leu Thr Lys Asn Gln Val Ser Leu Trp Cys Leu Val Lys
                485                 490                 495

Gly Phe Tyr Pro Ser Asp Ile Ala Val Glu Trp Glu Ser Asn Gly Gln
            500                 505                 510

Pro Glu Asn Asn Tyr Lys Thr Thr Pro Pro Val Leu Asp Ser Asp Gly
        515                 520                 525

Ser Phe Phe Leu Tyr Ser Lys Leu Thr Val Asp Lys Ser Arg Trp Gln
    530                 535                 540

Gln Gly Asn Val Phe Ser Cys Ser Val Met His Glu Ala Leu His Asn
545                 550                 555                 560

His Tyr Thr Gln Lys Ser Leu Ser Leu Ser Pro Gly Lys
                565                 570

## Description

### Technical Field

The present disclosure provides an antibody fragment specifically binding to PSMA, and particularly relates to a single-chain Fv (scFv) specifically binding to PSMA, a bispecific antibody containing the scFv, and a chimeric antigen receptor (CAR) and the preparation and the use thereof.

### Background

Prostatic cancer is the second leading cause of death in men, ranking only second to lung cancer. However, the current methods, for example, surgery, radiotherapy, chemotherapy and androgen deprivation therapy, have limited effects on those advanced cancers. Up to now, radical surgery is still the major treatment for prostatic cancer. Therefore, emerging precision medicine, particularly the precision medicine based on tumor-targeted antibodies is expected to improve the outcome for prostate cancer sufferers.

Prostate specific membrane antigen (PSMA) is mainly expressed by prostatic epithelial cells. In prostatic cancer, especially in poorly differentiated, metastatic, and hormone-resistant cancers, the expression of PSMA is markedly increased (Gregorakis AK, et al., (1998) Seminars in Urologic Oncology 16: 2-12; Silver, DA (1997) Clinical Cancer Research 3: 85-515). Low-level expression of PSMA has been found in extra-prostate tissues, such as small intestine, salivary glands, duodenum mucosa, proximal renal tubules and brain (Silver, DA (1997) Clinical Cancer Research 3: 85-515). PSMA is also expressed in the endothelial cells of capillaries in the peripheral and intratumor regions of some malignant tumors (including renal cell carcinoma and colorectal carcinoma), but not in the blood vessels of normal tissues. Moreover, it is reported that PSMA is associated with tumor angiogenesis (Silver, D.A. (1997) Clinical Cancer Research 3: 81-85). Recently, it has been proved that PSMA is expressed in the endothelial cells of tumor-associated neovasculature system in colorectal cancer , breast cancer, bladder cancer, pancreatic cancer, kidney cancer, and melanoma (Chang, S.S. (2004) Curr Opin Investig Drugs 5: 611-5), laying the foundation for developing prostatic cancer-targeted precision medicine.

### Summary

The present disclosure screens and obtains a series of high-affinity antibodies against PSMA by phage display technology. The inventor further finds that in these antibodies presented in the form of scFv, the amino acids at position 42 of the light chain (VL) has remarkable influence on the affinity; further, the protein yield of bispecific antibodies containing VL with a specific amino acids at position 42 has been significantly improved. The aPSMA scFv antibody fragment in the present disclosure may be used to construct bispecific antibodies, chimeric antigen receptors and the like, meeting the affinity requirements for aPSMA scFv in the field of tumor targeted therapy.

In one aspect, the present disclosure, provides an antibody fragment that specifically binds human PSMA, including:
A) a heavy chain variable region (VH) with the amino acid sequence as shown in SEQ ID NO: 2;
B) a light chain variable region (VL) with the amino acid sequence selected from the followings: SEQ ID NO: 6, SEQ ID NO: 8, SEQ ID NO: 10, SEQ ID NO: 12, SEQ ID NO: 14 or SEQ ID NO: 52.

In some embodiments, the antibody fragment that specifically binds PSMA is a single-chain Fv (scFv). In some embodiments, the PSMA-targeted scFv has a VH with the amino acid sequence shown in SEQ ID NO: 2 and a VL with the amino acid sequence shown in SEQ ID NO: 6. In some embodiments, the PSMA-targeted scFv has a VH with the amino acid sequence shown in SEQ ID NO: 2 and a VL with the amino acid sequence shown in SEQ ID NO: 8. In some embodiments, the PSMA-targeted scFv has a VH with the amino acid sequence shown in SEQ ID NO: 2 and a VL with the amino acid sequence shown in SEQ ID NO: 10. In some embodiments, the PSMA-targeted scFv has a VH with the amino acid sequence shown in SEQ ID NO: 2 and a VL with the amino acid sequence shown in SEQ ID NO: 12. In some embodiments, the PSMA-targeted scFv has a VH with the amino acid sequence shown in SEQ ID NO: 2 and a VL with the amino acid sequence shown in SEQ ID NO: 14. In some embodiments, the PMSA-targeted scFv has a VH with the amino acid sequence shown in SEQ ID NO: 2 and a VL with the amino acid sequence shown in SEQ ID NO: 52.

In some embodiments, the PSMA-targeted scFv has an amino acid sequence as shown in SEQ ID NO: 20. In some embodiments, the PSMA-targeted scFv has an amino acid sequence as shown in SEQ ID NO: 22. In some embodiments, the PSMA-targeted scFv has an amino acid sequence as shown in SEQ ID NO: 24. In some embodiments, the PSMA-targeted scFv has an amino acid sequence as shown in SEQ ID NO: 26. In some embodiments, the PSMA-targeted scFv has an amino acid sequence as shown in SEQ ID NO: 28. In some embodiments, the PSMA-targeted scFv has an amino acid sequence as shown in SEQ ID NO: 30. In some embodiments, the PSMA-targeted scFv has an amino acid sequence as shown in SEQ ID NO: 32. In some embodiments, the PSMA-targeted scFv has an amino acid sequence as shown in SEQ ID NO: 34. In some embodiments, the PSMA-targeted scFv has an amino acid sequence as shown in SEQ ID NO: 36. In some embodiments, the PSMA-targeted scFv has an amino acid sequence as shown in SEQ ID NO: 38. In some embodiments, the PSMA-targeted scFv has an amino acid sequence as shown in SEQ ID NO: 54. In some embodiments, the PSMA-targeted scFv has an amino acid sequence as shown in SEQ ID NO: 56.

In one aspect, the present disclosure provides a bispecific antibody, wherein the bispecific antibody including:
1) a first antigen binding domain specifically binding to PSMA, including:
   A) a heavy chain variable region (VH) having an amino acid sequence as shown in SEQ ID NO: 2; and
   B) a light chain variable region (VL) having an amino acid sequence selected from the following: SEQ ID NO: 6, SEQ ID NO: 8, SEQ ID NO: 10, SEQ ID NO: 12, SEQ ID NO: 14 or SEQ ID NO: 52, and
2) a second antigen binding domain.

In some embodiments, the first antigen binding domain of the bispecific antibody is a single-chain Fv (scFv) specifically binding to PSMA. In some embodiments, the first antigen binding domain of the bispecific antibody has an amino acid sequence as shown in SEQ ID NO: 20. In some embodiments, the first antigen binding domain of the bispecific antibody has an amino acid sequence as shown in SEQ ID NO: 22. In some embodiments, the first antigen binding domain of the bispecific antibody has an amino acid sequence as shown in SEQ ID NO: 24. In some embodiments, the first antigen binding domain of the bispecific antibody has an amino acid sequence as shown in SEQ ID NO: 26. In some embodiments, the first antigen binding domain of the bispecific antibody has an amino acid sequence as shown in SEQ ID NO: 28. In some embodiments, the first antigen binding domain of the bispecific antibody has an amino acid sequence as shown in SEQ ID NO: 30. In some embodiments, the first antigen binding domain of the bispecific antibody has an amino acid sequence as shown in SEQ ID NO: 32. In some embodiments, the first antigen binding domain of the bispecific antibody has an amino acid sequence as shown in SEQ ID NO: 34. In some embodiments, the first antigen binding domain of the bispecific antibody has an amino acid sequence as shown in SEQ ID NO: 36. In some embodiments, the first antigen binding domain of the bispecific antibody has an amino acid sequence as shown in SEQ ID NO: 38. In some embodiments, the first antigen binding domain of the bispecific antibody has an amino acid sequence as shown in SEQ ID NO: 54. In some embodiments, the first antigen binding domain of the bispecific antibody has an amino acid sequence as shown in SEQ ID NO: 56.

In some embodiments, the second antigen binding domain of the bispecific antibody binds to a specific receptor of T cells, wherein the specific receptor of T cells is preferably CD3. In some embodiments, the second antigen binding domain specifically binding to CD3 has a VH as shown in SEQ ID NO: 40 and a VL as shown in SEQ ID NO: 42.

In some embodiments, the bispecific antibody has sequences as shown in SEQ ID NO: 44 and SEQ ID NO: 46. In some embodiments, the bispecific antibody has sequences as shown in SEQ ID NO: 58 and SEQ ID NO: 60. In some embodiments, the bispecific antibody has sequences as shown in SEQ ID NO: 62 and SEQ ID NO: 64. In some embodiments, the bispecific antibody has sequences as shown in SEQ ID NO: 58 and SEQ ID NO: 66. In some embodiments, the bispecific antibody has sequences as shown in SEQ ID NO: 68 and SEQ ID NO: 64.

In one aspect, the present disclosure provides a chimeric antigen receptor (CAR), where the CAR comprises a scFv specifically binding to PSMA, a trans-membrane domain and an intracellular domain.

In some embodiments, the PSMA-targeted scFv of the CAR has a VH as shown in SEQ ID NO: 2 and a VL as shown in SEQ ID NO: 6. In some embodiments, the PSMA-targeted scFv of the CAR has a VH as shown in SEQ ID NO: 2 and a VL as shown in SEQ ID NO: 8. In some embodiments, the PSMA-targeted scFv of the CAR has a VH as shown in SEQ ID NO: 2 and a VL as shown in SEQ ID NO: 10. In some embodiments, the PSMA-targeted scFv of the CAR has a VH as shown in SEQ ID NO: 2 and a VL as shown in SEQ ID NO: 12. In some embodiments, the PSMA-targeted scFv of the CAR has a VH as shown in SEQ ID NO: 2 and a VL as shown in SEQ ID NO: 14. In some embodiments, the PSMA-targeted scFv of the CAR has a VH as shown in SEQ ID NO: 2 and a VL as shown in SEQ ID NO: 52. In some embodiments, the PSMA-targeted scFv of the CAR has an amino acid sequence as shown in SEQ ID NO: 20. In some embodiments, the PSMA-targeted scFv of the CAR has an amino acid sequence as shown in SEQ ID NO: 22. In some embodiments, the PSMA-targeted scFv of the CAR has an amino acid sequence as shown in SEQ ID NO: 24. In some embodiments, the PSMA-targeted scFv of the CAR has an amino acid sequence as shown in SEQ ID NO: 26. In some embodiments, the PSMA-targeted scFv of the CAR has an amino acid sequence as shown in SEQ ID NO: 28. In some embodiments, the PSMA-targeted scFv of the CAR has an amino acid sequence as shown in SEQ ID NO: 32. In some embodiments, the PSMA-targeted scFv of the CAR has an amino acid sequence as shown in SEQ ID NO: 34. In some embodiments, the PSMA-targeted scFv of the CAR has an amino acid sequence as shown in SEQ ID NO: 36. In some embodiments, the PSMA-targeted scFv of the CAR has an amino acid sequence as shown in SEQ ID NO: 38. In some embodiments, the PSMA-targeted scFv of the CAR has an amino acid sequence as shown in SEQ ID NO: 54. In some embodiments, the PSMA-targeted scFv of the CAR has an amino acid sequence as shown in SEQ ID NO: 56. In some embodiments, the CAR has an amino acid sequence as shown in SEQ ID NO: 50.

In one aspect, the present disclosure provides a polynucleotide which encodes the antibody fragment, bispecific antibody or CAR that specifically binds to PSMA in any one of the preceding embodiments. In some embodiments, the polynucleotide comprises a nucleotide sequence selected from the following group: 5, 7, 9, 11, 13, 19, 21, 23, 25, 27, 29, 31, 33, 35, 37, 39, 41, 43, 44, 45, 47, 49, 51, 53, 55, 57, 59, 61, 63, 65, and 67.

In one aspect, the present disclosure provides a vector containing the preceding polynucleotide.

In one aspect, the present disclosure provides a cell comprising the preceding vector.

In one aspect, the present disclosure provides a composition containing the antibody fragment, the bispecific antibody, the CAR, the polynucleotide, the vector, or the cell of any one of the preceding items, and the composition further comprising a pharmaceutically acceptable carrier.

In one aspect, the present disclosure provides a method for treating a subject suffering from a disease associated with PSMA expression, including administering to the subject an effective amount of the composition.

In one respect, the present disclosure provides a method for diagnosing a disease associated with PSMA expression in a mammal, and the method comprising: using the antibody fragment according to any one of claims 1-3 to detect the binding to human PSMA in a tissue sample separated from the mammal, and thus the specific binding of the antibody fragment to the human PSMA in the tissue sample is indicative of a disease associated with PSMA expression in the mammal.

### Brief Description of the Drawings

The drawings constituting the present application are used to provide a further understanding to the prevent disclosure. The exemplary embodiments and descriptions of the prevent disclosure are intended to explain the prevent disclosure, but not be construed as improper limitation to this disclosure. In the drawings:
FIG. 1 shows the ELISA result of aPSMA LH RM and RM2 monoclonal phage, wherein RM denotes the first round of panning, and RM2 denotes the second round of panning.
FIG. 2 shows the ELISA result of aPSMA HL monoclonal phage.
FIG. 3A shows the ELISA binding of an aPSMA scFv antibody with amino acid mutation at position 42 of VL to PSMA-his; and FIG. 3B shows the binding of a PSMA-targeted scFv-Fc antibody (on the 42nd amino acid mutation of a VL) to LnCap cells as detected by flow cytometry.
FIG. 4 shows the binding of bispecific antibodies to Jurkat T cells as detected by flow cytometry.
FIG. 5 shows the binding of bispecific antibodies to LnCap cells as detected by flow cytometry.
FIG. 6 shows the cytotoxicity of the bispecific antibody on LnCap cells by recruiting human T cells.
FIG. 7 shows the PK curve of the bispecific antibody in mice.

### Detailed Description of the Embodiments

### Examples

It should be noted that, in the case of no conflict, the embodiments of the present application are merely examples for illustration, and are not intended to limit the present disclosure in any way.

### Examples

### Example 1 Phage library screening

aPSMA HL (SEQ ID NO: 37) and aPSMA LH (SEQ ID NO: 39) were synthesized, and respectively cloned into the phagemid vector, subjected to random mutation with GeneMorph II random mutagenesis (Agilent), and transformed into XL1-blue competent cells. When OD600=0.6-0.8, the transformed XL1-blue cells were infected with helper phage M13K07. After 12 hours, the phage was collected and the phage titer (phage library capacity) was measured.

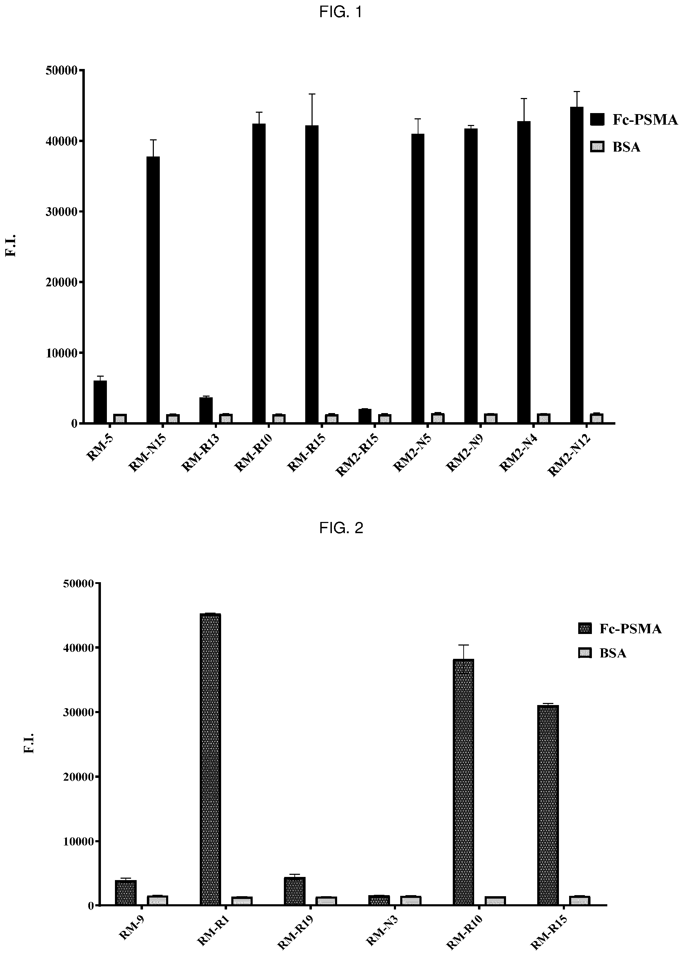

A microwell plate was coated with Fc-PSMA (300ng/well) and blocked by BSA, the preceding two phage libraries were subjected to 2-3 rounds of panning and affinity detection, and single clones were picked randomly from the panned plates and sent for sequencing. Sequencing result analysis was performed. The single clones with sequence enrichment or mutations in the CDR regions were subjected to phage packaging and affinity detection. The detection result was shown in FIG. 1.

FIG. 1 shows the affinity detection result of the aPSMA LH RM and aPSMA LH RM2 mutant libraries. It can be seen from the figure that except for aPSMA LH RM-R13 and aPSMA LH RM2-R15, the affinity of other clones is much higher than that of the original sequence; FIG. 2 shows the affinity detection result of the aPSMA HL RM mutant library, and only aPSMA HL RM-R1/R10/R15 has a higher affinity.

After analyzing the above-mentioned sequences with higher affinity, it is found that in the aPSMA LH RM, aPSMA LH RM2 and aPSMA HL RM mutant libraries, the clones with higher affinity have a same amino acid mutation at the 42nd position of VL.

**Table 1 Sequence Listing**

| | Description | Nucleotide sequence | Amino acid sequence |
|---|---|---|---|
| | | SEQ ID NO: | SEQ ID NO: |
| aPSMA VH | VH | 1 | 2 |
| aPSMA VL | VL | 3 | 4 |
| aPSMA VL (L42) | VL | 5 | 6 |
| aPSMA VL (H42) | VL | 7 | 8 |
| aPSMA VL (T42) | VL | 9 | 10 |
| aPSMA VL (S42) | VL | 11 | 12 |
| aPSMA VL (Q42) | VL | 13 | 14 |
| aPSMA HL | scFv | 15 | 16 |
| aPSMA LH | scFv | 17 | 18 |
| aPSMA HL (L42) | scFv | 19 | 20 |
| aPSMA HL (H42) | scFv | 21 | 22 |
| aPSMA HL (T42) | scFv | 23 | 24 |
| aPSMA HL (S42) | scFv | 25 | 26 |
| aPSMA HL (Q42) | scFv | 27 | 28 |
| aPSMA LH (Q42) | ScFv | 29 | 30 |
| aPSMA LH (S42) | ScFv | 31 | 32 |
| aPSMA LH (T42) | ScFv | 33 | 34 |
| aPSMA LH (H42) | ScFv | 35 | 36 |
| aPSMA LH (L42) | ScFv | 37 | 38 |
| aCD3 VH | VH | 39 | 40 |
| aCD3 VL | VL | 41 | 42 |
| aCD3-aPSMA | Chain-1 | 43 | 44 |
| | Chain-2 | 45 | 46 |
| aCD3 | Chain-1 | 43 | 44 |
| | Chain-2 | 47 | 48 |
| aPSMA CAR | PSMA-targeted chimeric antigen receptor (CAR) | 49 | 50 |
| aPSMA VL (K42) | VL | 51 | 52 |
| aPSMA HL (K42) | scFv | 53 | 54 |
| aPSMA LH (K42) | scFv | 55 | 56 |
| aCD3-aPSMA.2 | Chain-1 | / | 57 |
| | Chain-2 | / | 58 |
| aCD3-aPSMA.3 | Chain-1 | / | 59 |
| | Chain-2 | / | 60 |
| aCD3-aPSMA.4 | Chain-1 | / | 57 |
| | Chain-2 | / | 61 |
| aCD3-aPSMA.5 | Chain-1 | / | 62 |
| | Chain-2 | / | 60 |

### Example 2 Detection of the binding affinity of PSMA-targeted antibody

### 2.1 Cloning and expression of PSMA-targeted scFv-Fc antibody

aPSMA HL (SEQ ID NO: 15), aPSMA LH (SEQ ID NO: 17), aPSMA HL (L42) (SEQ ID NO: 19), aPSMA HL (H42) (SEQ ID NO: 21), aPSMA HL (T42) (SEQ ID NO: 23), aPSMA HL (S42) (SEQ ID NO: 25), aPSMA HL (Q42) (SEQ ID NO: 27) and aPSMA LH (Q42) (SEQ ID NO: 29) were cloned into the N-terminal of pFuse-hlgG1-Fc2 by traditional enzyme digestion and ligation methods, and subjected to sequencing for verification .

The constructed eukaryotic expression vector was transiently transfected into FreeStyle HEK293 cells (ThermoFisher), respectively: 28 ml FreeStyle HEK293 (3x10⁷ cell/ml) were seeded into a 125 ml cell culture flask, plasmids were diluted with 1 ml Opti-MEM (Invitrogen) and then added to 1 ml Opti-MEM containing 60 µl 293 Fectin (Invitrogen, Inc). After incubating at room temperature for 30 min, the plasmid-293fectin mixture was added to the cell culture , and then incubated at 125 rpm, 37°C, 5% CO2. Cell culture supernatant was collected at 48 h and 96 h after transfection, respectively, and purified by Protein A Resin (Genscript) according to the manufacturer's instruction. The purified proteins were analyzed by SDS-PAGE.

### 2.2 Affinity detection of PSMA-targeted scFv-Fc antibody aganist PSMA-His

PSMA-His(Acro) (100 ng/well) was coated in a 96-well plate and incubated at 4°C overnight. After blocked by PBST (0.5% Tween-20 in PBS) containing 2% skim milk powder for 1 h at room temperature, gradiently diluted scFv-Fc antibodies were added and incubated for 2 h at room temperature. After washed by PBST containing 2% skim milk powder for 4-5 times, anti-human Fc-HRP secondary antibody was added for incubation for 1 h at room temperature. After washed by PBST containing 2% skim milk powder for 4-5 times, TMB reagent (BioLegend, Cat. 421101) was added for color development, and readings at 650 nm (not termination) or 450 nm (termination) were recorded. Data was analyzed by nonlinear regression with specific binding model by Prizm Graphpad software.

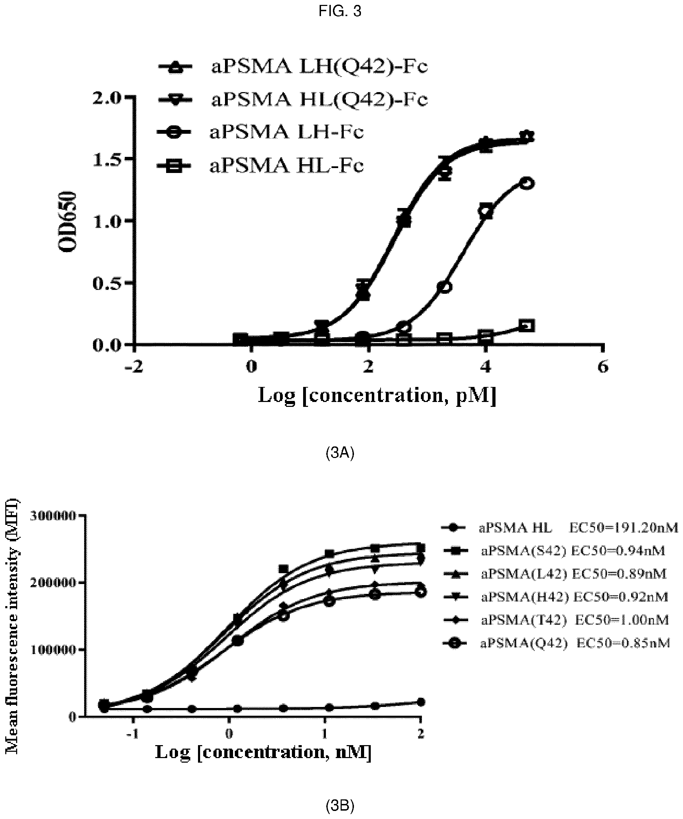

As shown in FIG. 3A, the aPSMA LH-Fc or aPSMA LH-Fc with wildtype VL has weak affinity agonist PSMA-his, while aPMSA HL (Q42)-Fc or aPSMA LH (Q42)-Fc with 42^{nd-}position amino acid mutation in VL has enhanced affinity to PSMA-his.

### 2.3 Binding analysis of the PSMA-targeted scFv-Fc antibody to LnCap cells by flow cytometry

LnCap cells were cultured in RPMI 1640 medium containing 10% FBS in a 5% CO2 incubator. 2^{*}10⁵ cells were washed for 3 times by pre-cooled PBS. After blocked by 2% FBS diluted by PBS, gradiently diluted antibodies (100 nM, gradiently diluted for 3 folds successively) were added and incubated for 1 hour at 4°C. Unbonded antibodies were washed away by 2% FBS. Mouse anti-human IgG Fc-APC (southern biotech) was added for 1 hour incubation at 4°C, and then subjected to flow cytometry after washed by 2% FBS. As shown in FIG. 3, the PSMA-targeted scFv-Fc with 42^{nd}-position amino acid mutation in VL has higher affinity aganist PSMA on the surface of LnCap cells, which is 190-220 times higher than that of PSMA-targeted scFv-Fc with wild type VL.

### Example 3 Bispecific antibody targeting PSMA and CD3

### 3.1 Construction and expression of bispecific antibodies targeting PSMA and CD3

Standard molecular biological methods were used to construct chain-1 and chain-2 containing different aCD3-aPMSA bispecific antibody as shown in Table 1 Sequence Listing, and subjected to sequencing for verification.

Eukaryotic expression vectors, containing the above-mentioned two chains, were verified by sequencing and transiently co-transfected into FreeStyle HEK293 cells (ThermoFisher) respectively: 28 ml FreeStyle HEK293 (3x10⁷ cell/ml) were seeded in a 125 ml cell culture flask, plasmids were diluted by 1 ml Opti-MEM (Invitrogen), and then added to 1 ml Opti-MEM containing 60 µl 293 Fectin (Invitrogen, Inc). After incubating at room temperature for 30min, and the plasmid-293fectin mixture was added to the cell culture, and incubated at 125 rpm, 372, 5% CO2. Cell culture supernatant was collected at 48 hours and 96 hours after transfection, respectively, and purified by Protein A Resin (Genscript) according to the manufacturer's instruction, The purified proteins were analyzed by SDS-PAGE detection, and the yield was calculated. The results showed that the yield of aCD3-aPSMA bispecific antibody (15-20mg/L) with 42^{nd-}position mutation in VL is significantly higher than that of aCD3-aPSMA bispecific antibody (2.5-5 mg/L) with a wild type VL.

### 3.2 In vitro activity evaluation of bispecific antibody targeting PSMA and CD3

### 3.2.1 Flow cytometry analysis on binding to Jurkat (CD3+) cells

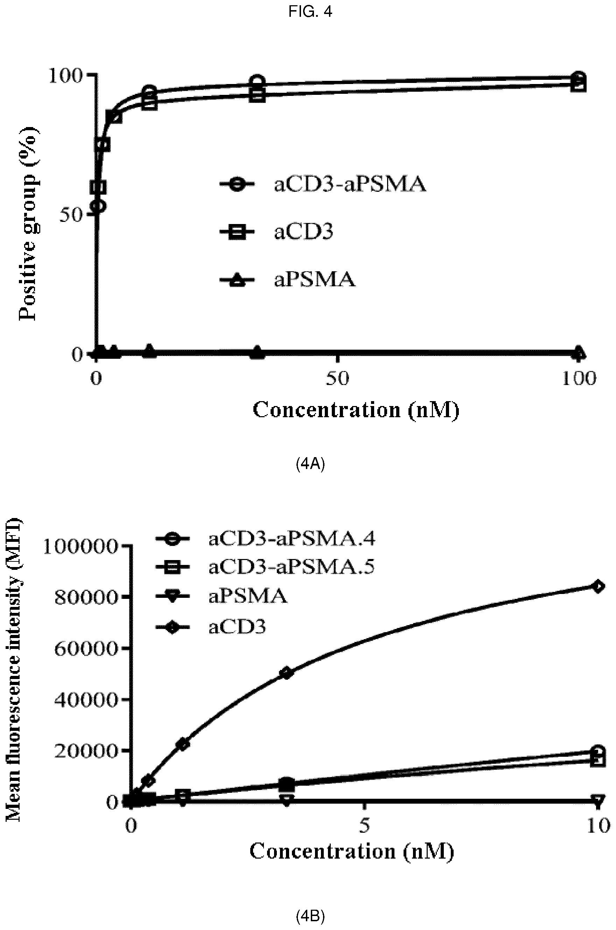

Jurkat cells were cultured in RPMI 1640 medium containing 10% FBS in a 5% CO2 incubator. 2x10⁵ cells were washed for 3 times with pre-cooled PBS. After blocked by PBS containing 2% FBS, bispecific antibody at concentrations of 100 nM, 33.3 nM, 11.1 nM, 3.7 nM, 1.2 nM or 0.4 nM was added and incubated for 1 hour at 4°C. Unbonded antibodies were washed by PBS containing 2% FBS, and mouse anti-human IgG Fc-APC (southern biotech) secondary antibody was added for 1 hour incubation at 4°C. After washing 3 times by PBS containing 2% FBS, flow cytometry was performed. As shown in FIG. 4, the bispecific antibody could effectively bind to Jurkat (CD3+) cells.

### 3.2.2 Flow cytometry analysis on binding to LnCap (PSMA+) cells

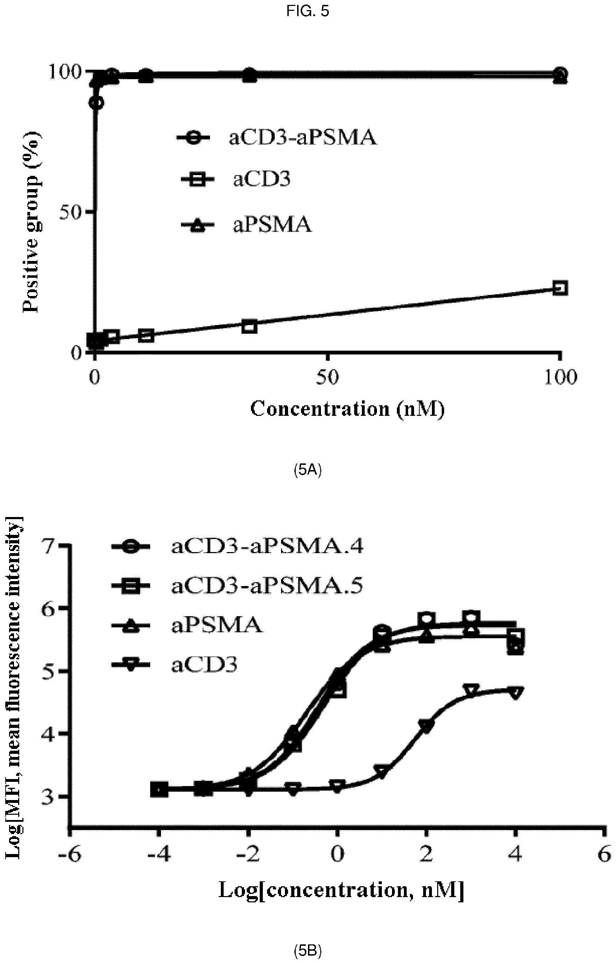

LnCap cells were cultured in RPMI 1640 medium containing 10% FBS in a 5% CO2 incubator. 2*10⁵ cells were washed for 3 times by pre-cooled PBS. After blocked by 2% FBS diluted by PBS, bispecific antibody at concentrations of 100 nM, 33.3 nM, 11.1 nM, 3.7 nM, 1.2 nM or 0.4 nM was added and incubated for 1 hour at 4°C. Unbonded antibodies were washed by 2% FBS, and mouse anti-human IgG Fc-APC (southern biotech) was added for incubation for 1 hour at 4°C. After washing by 2% FBS, flow cytometry was performed. As shown in FIG. 5, the bispecific antibody could effectively bind to LnCap cells.

**Table 2 EC₅₀ of the bispecific antibody binding to Jurkat and LnCap cells**

| EC₅₀ (nM) | aCD3-aPSMA | aCD3 | aPSMA |
|---|---|---|---|
| Jurkat | 0.51 | 0.51 | NA |
| LnCap | 0.05 | NA | 0.01 |

### 3.2.3 Detection of human T cell-dependent cytotoxicity

Peripheral blood was collected from healthy volunteer donor and peripheral blood mononuclear cells (PBMCs) were isolated by Ficoll-Hypaque (GE Healthcare) gradient centrifugation. The isolated PBMC was washed and incubated in RPMI medium containing 10% (vol/vol) FBS for 1 h to remove adherent cells. After activation by anti-CD3 and anti-CD28, cells were expanded in IL-2 for 10 days.

1x10⁴ LnCap cells (a RPMI medium containing 5% FBS) were mixed with activated PBMCs at a ratio of 1:5, and then incubated with bispecific antibody at different dilutions for 16 h at 37°C. The content of lactic dehydrogenase (LDH) in supernatant was detected by Cytotox-96 nonradioactive cytotoxicity assay kit (Promega). The value obtained from wells only containing target cells treated by the lysate was the Target Cell Maximum LDH Release Control; the value obtained from wells containing both effector cells and target cells treated by PBS (vehicle) was Spontaneous LDH Release Control. Color development was performed as the manufacturer's instruction, and absorbance at 490nm was recorded in BMG LAB TECH CLARIOstar microplate reader (Bio-Gene Technology Ltd.). cytotoxicity was calculated as follows: % cytotoxicity = (absorbance experimental - absorbance spontaneous average)/(absorbance maximum killing average - absorbance spontaneous average).

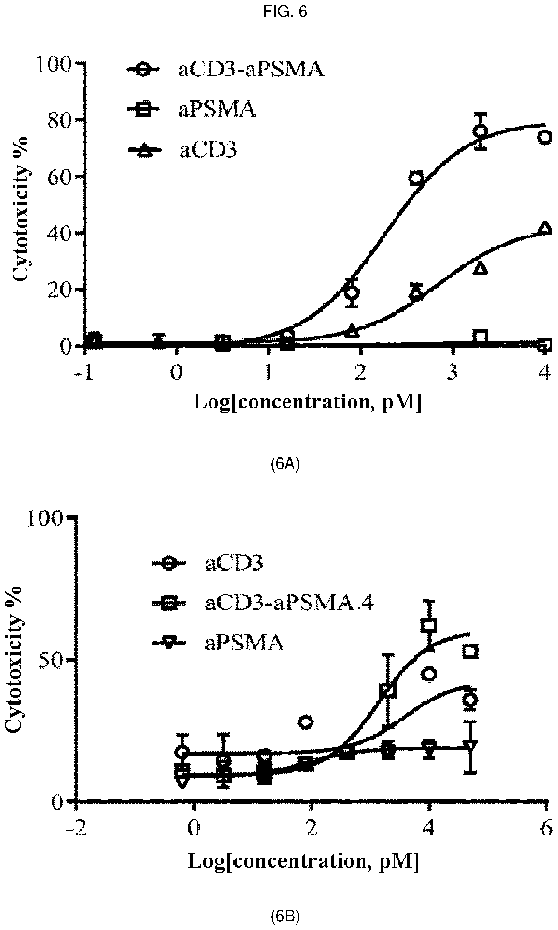

As shown in FIG. 6, aCD3-aPSMA bispecific antibody could effectively recruit T cells to exert killing effects on target cells LnCap when compared with the control aPSMA.

**Table 3 Killing of the bispecific antibody on the target cell LnCap via calling on PBMC**

| | | | |
|---|---|---|---|
| | aCD3-aPSMA | aCD3 | aPSMA |
| LDH Killing EC₅₀ (pM) | 184.4 | 712.3 | NA |
| LDH max killing (pM) | 79.79 | 42.76 | NA |

### 3.3 PK study on the PSMA and CD3-targeted bispecific antibody

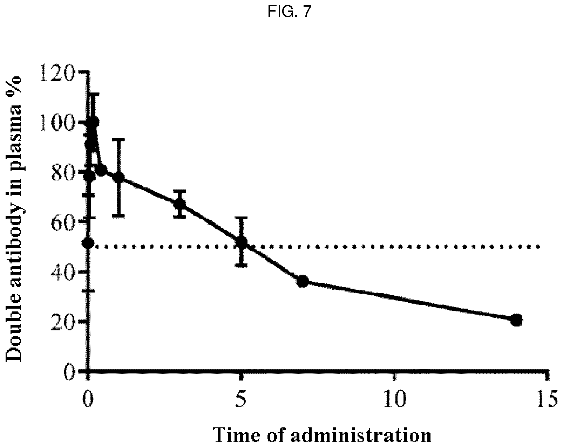

The bispecific antibody was intraperitoneally injected (I.P.) into C57 female mice (3 pieces/group, at a dose of 10 mg/kg). Whole blood was collected 30 min, 1 h, 2 h, 4 h, 10 h, 24 h, 3 d, 5 d, 7 d, and 14 d after injection. After centrifugation, plasma was collected and kept at -80ºC for further use. The bispecific antibody in the plasma was detected with reference to the Example 2.2. As shown in Fig.7, the half-life of the bispecific antibody in mice is about 5 d.

## Claims

1. An antibody fragment specifically binding to human PSMA, comprising:
A) a heavy chain variable region (VH) with an amino acid sequence as shown in SEQ ID NO: 2; and
B) a light chain variable region (VL) with an amino acid sequence selected from the followings: SEQ ID NO: 6, SEQ ID NO: 8, SEQ ID NO: 10, SEQ ID NO: 12, SEQ ID NO: 14 or SEQ ID NO: 52.

2. The antibody fragment according to claim 1, wherein the antibody fragment is a single-chain Fv (scFv).

3. The antibody fragment according to claim 2, wherein the scFv has an amino acid sequence as shown in SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 54 or SEQ ID NO: 56.

4. A bispecific antibody, the bispecific antibody comprising:
1) a first antigen binding domain specifically binding to PSMA, comprising:
A) a heavy chain variable region (VH) with an amino acid sequence as shown in SEQ ID NO: 2; and
B) a light chain variable region (VL) with an amino acid sequence selected from the followings: SEQ ID NO: 6, SEQ ID NO: 8, SEQ ID NO: 10, SEQ ID NO: 12, SEQ ID NO: 14 or SEQ ID NO: 52, and
2) a second antigen binding domain.

5. The bispecific antibody according to claim 4, wherein the first antigen binding domain is a scFv, preferably, the scFv of claim 3.

6. The bispecific antibody according to claim 4, wherein the second antigen binding domain binds to a specific acceptor molecule on T cells, and wherein the specific acceptor molecule on T cells is preferably CD3.

7. The bispecific antibody according to claim 6, wherein the second antigen binding domain specifically binding to CD3 has a VH as shown in SEQ ID NO: 40, and a VL as shown in SEQ ID NO: 42.

8. The bispecific antibody according to any one of claims 4-7, comprising a group consisting of two polypeptide chains bindable to CD3 and PMSA: SEQ ID NO: 44 and SEQ ID NO: 46; SEQ ID NO: 58 and SEQ ID NO: 60; SEQ ID NO: 62 and SEQ ID NO: 64; SEQ ID NO: 58 and SEQ ID NO: 66; and SEQ ID NO: 68 and SEQ ID NO: 64.

9. A chimeric antigen receptor (CAR), wherein the CAR comprises a scFv specifically binding to PSMA, a trans-membrane domain and an intracellular domain, and wherein the scFv comprises:
A) a heavy chain variable region (VH) with an amino acid sequence as shown in SEQ ID NO: 2; and
B) a light chain variable region (VL) with an amino acid sequence selected from the following: SEQ ID NO: 6, SEQ ID NO: 8, SEQ ID NO: 10, SEQ ID NO: 12, SEQ ID NO: 14 or SEQ ID NO: 52.

10. The CAR according to claim 9, having an amino acid sequence as shown in SEQ ID NO: 50.

11. A polynucleotide, encoding the antibody fragment according to any one of claims 1-3, or the bispecific antibody according to any one of claims 4-8, and or the CAR according to any one of claims 9-10.

12. The polynucleotide according to claim 11, having a nucleotide sequence as shown in SEQ ID NOS: 19, 21, 23, 25, 27, 29, 31, 33, 35, 37, 43, 45, 49, 51, 53, 55, 57, 59, 61, 63, 65 or 67.

13. A vector, comprising the polynucleotide according to claim 11 or 12.

14. A cell, comprising the polynucleotide according to any one of claims 11-12 or the vector according to claim 13.

15. The cell according to claim 14, wherein the cell is a T cell.

16. A composition, comprising the antibody fragment according to any one of claims 1-3, the bispecific antibody according to any one of claims 4-8, the CAR according to any one of claims 9-10, the polynucleotide according to any one of claims 11-12, the vector according to claim 13, or the cell according to any one of claims 14-15.

17. The composition of claim 16, further comprising a pharmaceutically acceptable carrier.

18. The composition of claim 16 or 17, used for chimeric antigen receptor T-cell (CAR-T) immunotherapy.

19. A method for treating a subject suffering from a disease associated with PSMA expression, comprising administering to the subject an effective amount of the composition according to any one of claims 16-18.

20. A method for diagnosing a disease associated with PSMA expression in a mammal, wherein the method comprises: using the antibody fragment according to any one of claims 1-3 to detect the human PSMA in tissue sample separated from the mammal, and thus the specific binding of the antibody fragment to the human PSMA in the tissue sample is indicative of a disease associated with PSMA expression in the mammal.
